# EUROPEAN PATENT SPECIFICATION

(11) **EP 4 103 635 B1**
(45) Date of publication and mention of the grant of the patent: **08.01.2025**
(21) Application number: 21704546.7
(22) Date of filing: 11.02.2021
(51) Int. Cl.: C08G 18/48, C08G 18/72, C08G 18/76, C08K 5/00, C09D 175/08

(54) **POLYURETHANE FOAM COMPOSITIONS WITH ANTIMICROBIAL PROPERTIES**
POLYURETHANSCHAUMZUSAMMENSETZUNG MIT ANTIMIKROBIELLEN EIGENSCHAFTEN
COMPOSITION DE MOUSSES DE POLYURÉTHANE AYANT DES PROPRIÉTÉS ANTIMICROBIENNES

(30) Priority: 12.02.2020 EP 20156828; 12.02.2020 US 202062975292 P
(43) Date of publication of application: 21.12.2022
(73) Proprietor: Sanitized AG, 3400 Burgdorf (CH)
(72) Inventor: CHAPERON, David, 3367 Thörigen (CH); CORBIERE, Tristan, 8051 Zürich (CH); MÜNGER, Cédric, 3608 Thun (CH)
(74) Representative: Jacobi, Markus Alexander
(86) International application number: PCT/EP2021/053368
(87) International publication number: WO 2021/160757

(56) References cited:
- WO-A1-2010/049316
- WO-A1-2019/016205
- WO-A2-2009/089346
- US-A1- 2016 120 176

## Description

The present invention relates to polyurethane compositions with antimicrobial properties, a polyurethane foam or coating comprising said polyurethane composition, an article prepared from said composition, foam or coating, a process for the preparation of said composition, the use of a complexing agent and an antimicrobial agent for the preparation of said composition or for imparting antimicrobial properties to a polyurethane composition, and a kit of components for preparing said composition.

Polyurethane (PU) foams find diverse uses, ranging e.g. from mattresses, home upholstery, car upholstery, to cosmetic/medical applicators for flexible foam types. For thermal or sound insulation applications in the construction or automobile segment other polyurethane foam types are used, so-called hard foams or rigid polyurethane foams.

WO 2019/016205 discloses the preparation of porous materials made of e.g. polyurethane, wherein the porous materials are used as thermal insulation material and in vacuum insulation systems.

Flexible foam densities are typically in the range from 40 to 1,000 kg/m³, more preferably from 40 to 100 kg/m³ for mattresses. Rigid foams used for insulation purposes present such density levels as well. Typically, these foams are prepared by adding a toluene diisocyanate (TDI), methylenediphenyl diisocyanate (MDI), or hexamethylene diisocyanate (HDI) component to a polyol component. Polyurethane reaction enables a linear chain extension, if the polyol component is a diol or a 3-dimensional branched chain extension if the polyol component is a triol. This reaction is called the curing process. Once the reaction is completed, the system is said to be cured. In general, catalysts are added to the composition to improve the kinetics of the polymerization reaction.

Blends of polyols with both a triol- and diol-component enable the optimization of desired properties, such as rigidity or elasticity of the cured PU foam.

The versatility of the polyurethane chemistry allows the preparation of other polyurethane systems such as adhesives/elastomers or of polyurethane based coatings.

Furthermore, reactive copolymer systems can be easily prepared from the appropriate di-isocyanate/isocyanate compounds and the appropriate polyol or block-copolymer polyol. Hybrid polymer-polyurethane systems can be prepared imparting advanced properties to the final cured polyurethane product. As a concluding remark, a polyurethane object can be produced in various forms as flexible or rigid foam with high or low density but also as solid elastomer.

Polyurethane articles can be obtained by mixing a part containing a polyol and a part containing an isocyanate. A urethane linkage is formed between the hydroxyl groups and isocyanate moieties. Typically, diisocyanates are used in combination with diols and/or triols to form polyurethane compounds, often with a slight excess of isocyanate groups over the hydroxyl groups.

The term polyol is mainly used for polyhydroxyl compounds such as polyester polyols or polyether polyols or polyether-ester polyols. Other types of polyhydroxyl compounds can also be used to form polyurethanes. Besides the formation of the urethane linkage other moieties may be formed, intentionally or not, such as urea linkages from the reaction of isocyanate with amine groups. Urea linkages may further react with an isocyanate to form biuret groups.

Reactive polyurethane based elastomers for joinery or sealing often consist of a polyurethane polymer chain terminated with at least two isocyanates groups. It can be obtained by adjusting the amounts of isocyanates versus polyol in a water-free and air-free environment. The presence of residual isocyanate groups allows the polymer to react with air moisture to form amine moieties, which can subsequently react with other polymer sidechains in their direct vicinity, releasing only minimal CO₂ amounts without expansion. As the reaction in that case is generally very slow, it is often desired to add dedicated catalysts to increase the kinetics. Such reactive systems are stored tightly closed and air-free in sealed cartridges or drums, as the air humidity is necessary to complete the crosslinking. Thermoplastic polyurethane can be obtained by a similar strategy by using diols and di-isocyanates only.

For polyurethane foams, the foaming process is enabled by the introduction of controlled amounts of water. Water reacts with isocyanate, forming the corresponding amine and the expansion gas CO₂. Introduction of further water may cause over-foaming, sometimes followed by foam collapsing.

However, with the aid of additives, the foaming characteristics may be adjusted to the desired level as far as it may be possible. Those additives comprise for example silicone-based surfactants to control the foam and acetone as a blowing agent. Furthermore, the molecular weight of the diol/triol plays a significant role on the physical properties of the cured foam. Shorter polyol molecules and a higher number of cross-linkable groups are preferred for preparing rigid foams. Rigid foams for most purposes require a high proportion of closed cells, which is often up to 95% of the total number of cells, whereas this is less desired for flexible foams.

Small molecule polyol mixtures imply mostly a higher hydroxyl number. Here the reactive parts can be mixed through a static or a dynamic mechanical mixing unit that is commonly used involving 2 reactive components or in a bucket using for example a simple paint stirrer for a limited time. For both methods, the foaming process is intended to start after the mixing and after or during having it poured (also known as PIP "Pour-in-Place") or sprayed.

Slabstock flexible foams are commonly produced in a continuous manner. The mixtures are poured in the liquid state or as a liquid dispersion directly onto a conveyor belt or similar surfaces, where the foam can expand freely during travel. Later along the conveyor, the obtained foam can be cut into smaller sections and allowed to complete the curing process, and then cooled down e.g. for 24 hours before being further segmented into almost any desired shapes. Further rigid or flexible foams production such as imprinting of uncured conveyed foam or open or sealed mold casting methods are industrially established as well. Post-processing of foams other than cutting the foams does exist such as flame lamination.

The fundamentals to those technologies are explained e.g. in "A Handbook of Polymer Foams" (David Eaves, 2004, Smithers Rapra publishing) and in "Szycher,s Handbook of Polyurethanes" (Michael Szycher, 2012, 2. Edition, CRC Press).

A broad number of additives is described in both manuscripts that find an implementation in actual industrial polyurethane formulations, such as e.g. flame retardants, light stabilizers, antioxidants, pigments, dyes, catalysts, blowing agents, and surfactants.

Microorganisms - bacteria, fungi, viruses and others - can lead to problems of health, odor formation, and often to the deterioration and destruction of polymer materials. For these reasons, antimicrobial substances such as e.g. fungicides, levuricides, bacteriocides and/or viricides are used. Polyurethane foam is especially susceptible to degradation by fungi, which results in changes in material specifications, staining as well as in the formation of unpleasant odors.

Therefore, there is a need for improved polyurethane foams which are resistant against microorganisms.

On the one hand, fungal and bacterial resistance is an essential aspect of lifetime of a commercial good. On the other hand, having a material which is locally showing antimicrobial activity is highly desirable for some specific applications, such as wound healing pads/dressing.

Antimicrobial properties of polyurethane foam are generally obtained by admixture or subsequent addition of antimicrobial substances, such as silver-based components, as for instance described in EP-A 2720538A or anti-microbial powders, such as sodium pyrithione dispersed in plasticizers as described in US 6294589. WO 2010/049316 discloses compositions containing antimicrobials in a hybrid network based on polyurethane.

Wound dressing brand products like PolyMem^{®} or Mepilex^{®} Ag incorporate silver as the active ingredient. Other brand products like Kendall^{®} AMD wound dressings are impregnated with PHMB (polyhexamethylene biguanide). Industrial foamed products containing antimicrobial agents can be purchased e.g. from many furniture resellers. Even 10,10,-oxybisphenoxyarsine (OBPA) is still found in some polyurethane foam applications, e.g. for household furniture.

Moreover, in industry, the usage of isothiazolinone components such as octyl-isothiazolinone (OIT) or benzisothiazolinone (BIT) or of zinc pyrithione are common for the preparation of antimicrobial polyurethane systems.

Antimicrobial properties can also be imparted to other polyurethane systems, like non-foamed polyurethane films, with the aid of silver-based components as described in EP-A 1385477.

JP-A 2009-533141 describes a wound dressing made of multiple layers containing at least one layer loaded with a biguanide disinfectant compound or silver or copper-based antimicrobials and a layer loaded with at least one chelating agent. Wound dressings of many types including polyester as fibers or films and polyurethanes as films or foams are being depicted. Silver and copper ions are often subjected to oxidation or photooxidation when in the wet phase or in contact to air, leading to the discoloration of the article. This oxidation also causes a drop of antimicrobial performance. The chelating agent stabilizes metal ions such as copper and silver by forming the corresponding complexes in solution. This stabilization enables a better availability of the ions in the wet phase and reduces the probability of discoloration caused by air- or photooxidation.

Combinations of metal ions and complexing agents used for antimicrobial purposes are disclosed e.g. in US 5688516, EP-A 2720538, WO 2004/007595, and US 2012/0322903. However, the use of complexing agents during the preparation of polyurethane may lead to inactivation of the catalysts in the polyurethane polymerization reaction, which in turn may lead to reduced quality of the final products. Therefore, there is a need for further approaches for imparting anti-microbial properties to polyurethanes.

The incorporation of quaternary ammonium moieties into polyurethane polymer backbone or as end groups has been shown to lead to antimicrobial properties of polyurethanes in WO 2007/068890, US 2016/0120176 and US 7459167. The advantage of such antimicrobial treatment consists in the reduced leaching tendency of the antimicrobial agents, as they are covalently bound to the polymer. WO 2015/002786 describes polymeric foams that are impregnated with an aqueous solution of quaternary ammonium salts after the preparation of the foam, and then dried.

WO 2009/089346 discloses disinfectant compositions comprising water-insoluble quaternary ammonium polymers, which can be e.g. impregnated into hydrophilic polyurethane foams or wound dressings.

These approaches involve specific reactive compounds that enable the covalent attachment of the ammonium moieties to the polyurethane and include additional reaction steps during the preparation of the foam or additional processing steps after the preparation of the foam, which both leads to higher procedural complexity and costs, and potentially to reduced process efficiency. There is therefore a need for a more efficient way of imparting antimicrobial properties to polyurethane articles, especially wherein the antimicrobial agents do not leach from the articles.

It has surprisingly been found that combinations of certain complexing agents and organic antimicrobially active compounds are capable of effectively imparting antimicrobial, especially anti-fungal properties on polyurethane articles without significant influence on the quality of the articles. In particular, it has been found that these complexing agents and organic antimicrobially active compounds show a cooperative effect with regard to antimicrobial efficacy. These compounds can be added to the polyurethane during its preparation process without the need for additional reaction steps or processing steps.

In the context of this invention, the term "cooperative effect" is to be understood analogously to the term "synergistic effect", which is typically used if two or more active ingredients show improved activity in comparison to the activity of the individual components or the mere addition of their individual activities. Thus, if a complexing agent and an organic antimicrobially active compound show a cooperative effect, this means that the complexing agent enhances the efficacy of the organic antimicrobially active compound.

One aspect of the present invention is a polyurethane composition (P) comprising:
a) from 80 to 99.9% by weight, based on the total weight of the polyurethane composition (P), of at least one polyurethane component (A);
b) from 0.05 to 1.5% by weight (500 to 15,000 ppm), based on the total weight of the polyurethane composition (P), of at least one complexing agent (B);
c) from 0.005 to 5% by weight (50 to 50,000 ppm), based on the total weight of the polyurethane composition (P), of at least one organic antimicrobially active component (C) comprising at least one nitrogen atom;
d) optionally up to 15% by weight, based on the total weight of the polyurethane composition (P), of one or more polymers (D) different from the at least one polyurethane component (A); and
e) optionally up to 5% by weight, based on the total weight of the polyurethane composition (P), of one or more additives (E);
wherein the at least one complexing agent (B) is selected from the group consisting of organic compounds having at least two functional moieties, of which at least one functional moiety is a carboxylic acid moiety or a salt thereof.

These polyurethane compositions (P) preferably lead to a cooperative effect of the components (B) and (C). The individual components are described below in detail.

Preferably, the polyurethane composition (P) consists of the at least one polyurethane component (A), the at least one complexing agent (B), the at least one organic antimicrobially active component (C) comprising at least one nitrogen atom, optionally the one or more polymers (D) and optionally the one or more additives (E).

In one embodiment, the polyurethane composition (P) comprises at least one complexing agent (B) selected from the group consisting of ethylenediamine tetraacetic acid (EDTA) or salts thereof, glycolic acid or salts thereof, malic acid or salts thereof, tartaric acid or salts thereof, lactic acid or salts thereof, citric acid or salts thereof, N-(1-carboxylato-ethyl)-iminodiacetic acid or salts thereof, nitrilotriacetic acid or salts thereof.

In one embodiment, the polyurethane composition (P) comprises at least one complexing agent (B) selected from ethylenediamine tetraacetic acid (EDTA) or salts thereof, preferably the disodium salt thereof or the tetrasodium salt thereof.

In one embodiment, the polyurethane composition (P) comprises at least one organic antimicrobially active component (C) comprising at least one nitrogen atom is selected from the group consisting of quaternary ammonium salts or primary, secondary, tertiary amines having at least one C₈-C₃₀ hydrocarbon moiety attached to the nitrogen atom, N-oxides of tertiary amines having at least one C₈-C₃₀ hydrocarbon moiety attached to the nitrogen atom, tertiary amines having a C₈-C₃₀ hydrocarbon moiety and two aminoalkyl moieties attached to the tertiary nitrogen atom, and polymers having repeating units comprising quaternary ammonium moieties or amine moieties.

In one embodiment, the polyurethane composition (P) comprises at least one antimicrobially active component (C) selected from the group consisting of quaternary ammonium salts having at least one aliphatic C₈-C₃₀ hydrocarbon moiety and at least one alkoxylated silylalkyl moiety attached to the nitrogen atom, N-oxides of tertiary amines having at least one C₈-C₃₀ hydrocarbon moiety attached to the nitrogen atom, quaternary ammonium salts having at least two aliphatic C₈-C₃₀ hydrocarbon moieties attached to the nitrogen atom, quaternary ammonium salts having at least one aliphatic C₈-C₃₀ hydrocarbon moiety and at least one aromatic C₆-C₁₄ hydrocarbon moiety attached to the nitrogen atom, poly(diallyldimethyl-ammonium chloride), poly(dialkylamine-co-epichlorohydrine), C₈-C₃₀ alkylamine dipropylenediamine and poly(ethylene imine).

In one embodiment, the polyurethane composition (P) comprises at least one antimicrobially active component (C) selected from quaternary ammonium salts having at least one aliphatic C₈-C₃₀ hydrocarbon moiety and at least one alkoxylated silylalkyl moiety attached to the nitrogen atom, preferably C₈-C₃₀-alkyldimethyl(3-trialkoxysilyl)propyl ammonium salts, more preferably dimethyltetradecyl(3-(trimethoxysilyl)propyl)ammonium chloride or dimethyloctadecyl(3-(trimethoxysilyl)propyl)ammonium chloride.

In one embodiment, the polyurethane composition (P) comprises at least one complexing agent (B) which is present in an amount of from 1,000 to 12,000 ppm, preferably in an amount of from 1,500 to 10,000 ppm, more preferably in an amount of from 2,000 to 8,000 ppm, based on the total weight of the polyurethane composition (P).

In one embodiment, the polyurethane composition (P) comprises at least one organic antimicrobially active component (C), comprising at least one nitrogen atom, which is present in amounts of:
i) 250 to 8,000 ppm, preferably 500 to 6,000 ppm, more preferably 625 to 5,000 ppm, based on the total weight of the polyurethane composition (P), and is dimethyltetradecyl(3-(trimethoxysilyl)propyl)ammonium chloride;
ii) 250 to 4,000 ppm, preferably 500 to 3,000 ppm, more preferably 625 to 2,500 ppm, based on the total weight of the polyurethane composition (P), and is dimethyloctadecyl(3-(trimethoxysilyl)propyl)ammonium chloride;
iii) 400 to 40,000 ppm, preferably 600 to 32,000 ppm, more preferably 800 to 12,000 ppm, even more preferably 1000 to 8,000 ppm, based on the total weight of the polyurethane composition (P), and is selected from quaternary ammonium salts having at least two aliphatic C₈-C₃₀ hydrocarbon moieties attached to the nitrogen atom, preferably didecyldimethyl ammonium chloride;
iv) 1,500 to 10,000 ppm, preferably 2,000 to 8,000 ppm, more preferably 2,500 to 5,000 ppm, based on the total weight of the polyurethane composition (P), and is selected from quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride;
v) 2,500 to 25,000 ppm, preferably 4,000 to 12,000 ppm, more preferably 5,000 to 10,000 ppm, based on the total weight of the polyurethane composition (P), and is selected from quaternary ammonium salts having one C₈-C₃₀ hydrocarbon moiety attached to the nitrogen atom, preferably myristyltrimethylammonium chloride; or
vi) 1,500 to 15,000 ppm, preferably 2,000 to 10,000 ppm, more preferably 2,500 to 8,000 ppm, based on the total weight of the polyurethane composition (P), and is selected from C₈-C₃₀ alkylamine dipropylenediamines, preferably laurylamine dipropylenediamine.

In one embodiment, the polyurethane composition (P) comprises:
i) 1,000 to 12,000 ppm, preferably 2,000 to 10,000 ppm, based on the total weight of the polyurethane composition (P), of EDTA or a salt thereof as complexing agent (B) and 1,000 to 1,500 ppm, based on the total weight of the polyurethane composition (P), of dimethyloctadecyl(3-(trimethoxysilyl)-propyl)ammonium chloride as organic antimicrobially active component (C);
ii) 3,000 to 12,000 ppm, preferably 5,000 to 10,000 ppm, based on the total weight of the polyurethane composition (P), of EDTA or a salt thereof as complexing agent (B) and 1,000 to 6,000 ppm, preferably 2,000 to 5000 ppm, based on the total weight of the polyurethane composition (P), of quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride as organic antimicrobially active component (C);
iii) 1,000 to 12,000 ppm, preferably 2,000 to 10,000 ppm, based on the total weight of the polyurethane composition (P), of EDTA or a salt thereof as complexing agent (B) and 8,000 to 25,000 ppm, preferably 10,000 to 20,000 ppm, based on the total weight of the polyurethane composition (P), of quaternary ammonium salts having one C₈-C₃₀ hydrocarbon moiety attached to the nitrogen atom, preferably myristyltrimethylammonium chloride as organic antimicrobially active component (C); or
iv) 3,000 to 12,000 ppm, preferably 5,000 to 10,000 ppm, based on the total weight of the polyurethane composition (P), of EDTA or salts thereof as complexing agent (B) and 1,500 to 15,000 ppm, preferably 2,500 to 10,000 ppm, based on the total weight of the polyurethane composition (P), of C₈-C₃₀ alkylamine dipropylenediamine, preferably laurylamine dipropylenediamine as organic antimicrobially active component (C).

The invention also relates to a polyurethane foam or a coating comprising the polyurethane composition (P) as described above.

A further aspect of the invention relates to an article, preferably a mattress, pillow, cushion or wound dressing, prepared from the polyurethane composition (P) as described above.

The invention also relates to a process for preparing the polyurethane composition (P) as described above, comprising the steps:
I) providing at least one polyol having at least two, preferably two or three hydroxyl moieties and at least one polyisocyanate having at least two, preferably two isocyanate moieties;
II) providing at least one complexing agent (B) and at least one organic antimicrobially active component (C) as described in any one of claims 1 to 9;
III) optionally providing at least one catalyst for promoting the formation of urethane moieties from the hydroxyl groups of the polyol and the isocyanate groups of the polyisocyanate;
IV) optionally providing one or more additives (E);
V) mixing the at least one polyol, the at least one complexing agent (B) and the at least one organic antimicrobially active component (C) to obtain a mixture (M);
VI) admixing the at least one polyisocyanate to the mixture (M);
VII) allowing the at least one polyol to react with the at least one polyisocyanate in order to obtain the polyurethane composition (P) according to any of claims 1 to 9;
wherein the optional catalyst and/or the optional one or more additives (E), if present, may be added to the mixture (M) before, after or together with the at least one polyisocyanate.

One further aspect is the use of a combination of at least one complexing agent (B) and at least one organic antimicrobially active component (C) and optionally one or more additives (E) as described above for preparing an antimicrobial polyurethane composition.

The invention also covers the use of a combination of at least one complexing agent (B) and at least one organic antimicrobially active component (C) and optionally one or more additives (E) as described above for providing antimicrobial properties on a polyurethane composition.

One further aspect is a kit of components for preparing a polyurethane composition or a polyurethane article, comprising at least one polyol having at least two, preferably two or three hydroxyl moieties; at least one polyisocyanate having at least two, preferably two isocyanate moieties; at least one complexing agent (B) as described above; at least one antimicrobially active component (C) as described above; and optionally one or more additives (E) as described above.

The components are described in more detail in the following:

### Polyurethane component (A):

In general, the polyurethane component (A) can be any type of polyurethane component, such as those known in the literature. For example, the polyurethane component (A) may be a linear or branched polyurethane useful for preparing polyurethane films such as polyurethane dispersion films, viscoelastic polyurethane foams, flexible polyurethane foams, rigid or hard polyurethane foams or thermoplastic polyurethane moldings.

Preferably, the polyurethane component (A) is obtainable from at least one polyol having at least two, preferably two or three hydroxyl moieties and at least one polyisocyanate having at least two, preferably two isocyanate moieties.

The at least one polyol may be any type of polyol, e.g. a polyhydroxyalkane, such as ethylene glycol, propylene glycol or glycerol, a polyester polyol, such as a polyester diol or a polyester triol, a polyether polyol, such as a polyether diol or a polyether triol, a polyether-ester polyol, such as a polyether-ester diol or a polyether-ester triol, or a mixture of any of the above.

Also, the polyisocyanate may be any type of polyisocyanate, e.g. an aryl diisocyanate such as toluene diisocyanate (TDI), an alkylenediaryl diisocyanate such as methylenediphenyl diisocyanate (MDI), an alkylene diisocyanate, such as hexamethylene diisocyanate (HDI), or a mixture of any of the above.

Preferably, the polyurethane component (A) is obtainable from a polyester diol, a polyester triol, a polyether diol, a polyether triol, a polyether-ester diol, a polyether-ester triol, or a mixture of any of the above as the at least one polyol, and toluene diisocyanate (TDI), methylenediphenyl diisocyanate (MDI), hexamethylene diisocyanate (HDI) or a mixture of any of the above as the at least one polyisocyanate.

The polyurethane component (A) is generally present in the polyurethane composition (P) in an amount of from 80 to 99.9% by weight, preferably from 83 to 99.85% by weight, more preferably from 95 to 97.5% by weight, based on the total weight of the polyurethane composition (P).

### Complexing agent (B):

In general, the complexing agent (B) is selected from the group consisting of organic compounds having at least two functional moieties, of which at least one functional moiety is a carboxylic acid moiety or a salt thereof. The term "functional moiety" in this context means that the moiety is functional in terms of forming complexes. Specifically, it means that the moiety contains at least one electron lone pair that can be active as a Lewis base.

For example, the functional moieties can be derived from amine-moieties, hydroxyl-moieties, carbonyl-moieties, carboxylic acid moieties or salts thereof.

For example, the at least one complexing agent (B) can be selected from the group consisting of ethylenediamine tetraacetic acid (EDTA) or salts thereof, glycolic acid or salts thereof, malic acid or salts thereof, tartaric acid or salts thereof, lactic acid or salts thereof, citric acid or salts thereof, N-(1-carboxylato-ethyl)-iminodiacetic acid or salts thereof, nitrilotriacetic acid or salts thereof.

Preferably, the at least one complexing agent (B) is selected from ethylenediamine tetraacetic acid (EDTA) or salts thereof.

More preferably, the at least one complexing agent (B) is the disodium salt of EDTA, the tetrasodium salt of EDTA or a mixture thereof. The salts can be used as anhydrous compounds or as their hydrates.

The at least one complexing agent (B) is generally present in the polyurethane composition (P) in an amount of from 500 to 15,000 ppm, preferably of from 1,000 to 12,000 ppm, more preferably from 1,500 to 10,000 ppm, even more preferably from 2,000 to 8,000 ppm, based on the total weight of the polyurethane composition (P).

### Organic antimicrobial active component (C):

In general, the at least one organic antimicrobially active component (C) may be any organic antimicrobially active component comprising at least one nitrogen atom, such as organic amines having at least one C₈-C₃₀ hydrocarbon moiety attached to the nitrogen atom and their derivatives, or polymers having repeating units comprising amine moieties or their derivatives.

For example, the at least one organic antimicrobially active component (C) may be selected from the group consisting of quaternary ammonium salts having at least one C₈-C₃₀ hydrocarbon moiety attached to the nitrogen atom, primary, secondary or tertiary amines having at least one C₈-C₃₀ hydrocarbon moiety attached to the nitrogen atom, N-oxides of tertiary amines having at least one C₈-C₃₀ hydrocarbon moiety attached to the nitrogen atom, tertiary amines having a C₈-C₃₀ hydrocarbon moiety and two amino-alkyl moieties attached to the tertiary nitrogen atom, and polymers having repeating units comprising quaternary ammonium moieties or amine moieties.

Preferably, the at least one antimicrobially active component (C) is selected from the group consisting of quaternary ammonium salts having at least one aliphatic C₈-C₃₀ hydrocarbon moiety and at least one alkoxylated silylalkyl moiety attached to the nitrogen atom, N-oxides of tertiary amines having at least one C₈-C₃₀ hydrocarbon moiety attached to the nitrogen atom, quaternary ammonium salts having at least two aliphatic C₈-C₃₀ hydrocarbon moieties attached to the nitrogen atom, quaternary ammonium salts having at least one aliphatic C₈-C₃₀ hydrocarbon moiety and at least one aromatic C₆-C₁₄ hydrocarbon moiety attached to the nitrogen atom, poly(diallyldimethyl-ammonium chloride), poly(dialkylamine-co-epichlorohydrine), C₈-C₃₀ alkylamine dipropylenediamine and poly(ethylene imine).

More preferably, the at least one antimicrobially active component (C) is selected from the group consisting of quaternary ammonium salts having at least one aliphatic C₈-C₃₀ hydrocarbon moiety and at least one alkoxylated silylalkyl moiety attached to the nitrogen atom, in particular C₈-C₃₀-alkyldimethyl(3-trialkoxysilyl)propyl ammonium salts, especially dimethyltetradecyl(3-(trimethoxysilyl)propyl)ammonium chloride or dimethyloctadecyl(3-(trimethoxysilyl)propyl)-ammonium chloride.

A non-exclusive list of exemplary components (C), suitable for the compositions of the invention is given hereafter as examples:
dimethyloctadecyl[3-(trimethoxysilyl)propyl]ammonium chloride [CAS Nr. 27668-52-6], heptadecyl-dimethyl-(3-trimethoxysilylpropyl)azanium chloride, hexadecyl-dimethyl-(3-trimethoxysilylpropyl)azanium chloride, pentadecyl-di-methyl-(3-trimethoxysilylpropyl)azanium chloride, tetradecyldimethyl(3-trimethoxysilylpropyl)ammonium chloride [CAS Nr. 41591-87-1], tridecyl-dimethyl-(3-trimethoxysilylpropyl)azanium chloride, dodecyldimethyl(3-(triethoxysilyl) propyl)ammonium chloride [CAS Nr. 62077-89-8], trimethoxy-silylpropyldidecylmethylammonium chloride [CAS Nr. 68959-20-6], N,N-dibutyl-N-(3-(trimethoxysilyl)propyl)butan-1-aminium chloride [CAS Nr. 143203-33-2], dimethyloctadecyl(3-(triethoxysilyl)propyl)ammonium chloride [CAS Nr. 62117-57-1], hexadecyldimethyl(3-(triethoxysilyl)propyl)ammonium chloride[CAS Nr. 94134-21-1], dimethyl-pentadecyl-(3-triethoxysilylpropyl) azanium chloride, dimethyl-tetradecyl-(3-triethoxysilylpropyl)azanium chloride, dodecyldi-methyl(3-(triethoxysilyl)propyl)ammonium chloride [CAS Nr. 62077-89-8], di-methyl-(3-trimethoxysilylpropyl)-undecylazanium chloride , decyl-dimethyl-(3-triethoxysilylpropyl)azanium chloride, N,N-dimethyl-N-[3-(trimethoxysilyl)propyl]octan-1-aminium chloride [CAS Nr. 83354-12-5], benzyldimethyl(3-(triethoxysilyl)propyl)ammonium chloride [CAS Nr. 85391-03-3], benzyl-dimethyl-(3-trimethoxysilylpropyl)azanium chloride, trimethyl (octadecyl)azanium chloride [CAS Nr. 112-03-8], heptadecyl(trimethyl)azanium chloride, hexadecyltrimethylammonium chloride [CAS Nr. 112-02-7], 1-pentadecanaminium,n,n-trimethyl-, chloride [CAS Nr. 73163-54-9], tetradecyltrimethylammonium chloride [CAS Nr. 4574-04-3], N,N,N-trimethyltridecan-1-aminium chloride [CAS Nr. 19727-46-9], dodecyltrimethylammonium chloride [CAS Nr. 112-00-5], trimethyl(undecyl)azanium chloride [CAS Nr: n.a.], decyltrimethylammonium chloride [CAS Nr. 10108-87-9], didecyl dimethyl ammonium chloride [CAS Nr. 7173-51-5], trimethyl-[10-(trimethylazaniumyl)decyl]azanium [CAS Nr. 156-74-1], di-methyl(dioctadecyl)azanium chloride [CAS Nr. 107-64-2], octyltrimethylammonium chloride [CAS Nr. 10108-86-8], bis(3-aminopropyl)dimethylammonium chloride [CAS Nr. n.a.], C8-18-alkydimethylbenzyl ammonium chlorides [CAS Nr. 63449-41-2], myristyltrimethylammonium chloride [CAS Nr. 4574-04-3], (Diisobutylphenoxyethoxyethyl)dimethylbenzylammonium chloride [CAS Nr. 121-54-0], stearyltrimethylammonium chloride [CAS Nr. 112-03-08], cetrimonium bromide [CAS Nr. 57-09-0], polyquaternium 6 oder polydadmac [CAS Nr. 26062-79-3 ], 1-decanaminium, N-decyl-N-(2-hydroxyethyl)-N-methyl-, propanoate [CAS Nr. 107879-22-1], and N-alkyl-N-benzyl-N,N-dimethylammonium chloride [CAS Nr. 8001-54-5], benzethonium chloride [CAS Nr. 121-54-0], N-butyl-N-[3-(trimethoxysilyl)propyl]butan-1-amine [CAS Nr. 40835-31-2], N,N-didodecyldodecan-1-amine [CAS Nr. 102-87-4], tri-N-decylamine [CAS Nr. 1070-01-5], trihexylamine [CAS Nr. 102-86-3], diethyldodecylamine [CAS Nr. 4271-27-6], n,n-diethylhexadecylamine [CAS Nr. 250-403-2], N,N-dipropyl-dodecyl amine [CAS Nr. n.a.], bis(3-aminopropyl)amine [CAS Nr. 56-18-8], bis (3-aminopropyl) dodecylamine [CAS Nr. 2372-82-9], N-benzyl-N-ethylethanamine [CAS Nr. 772-54-3], N-benzyl-N-methylethanamine [CAS Nr. 4788-37-8], n,n-dimethyl-1-phenylmethanamine [CAS Nr. 103-83-3], N-benzyl-N-methyl-1-phenylmethanamine [CAS Nr. 102-05-6], 1,6-Bis(N5-[p-chlorophenyl]-N1-biguanido)hexane [CAS Nr. 55-56-1] N,N-dibenzyl-1-phenylmethanamine [CAS Nr. 620-40-6], 2-(2-phenylethylamino)ethanol [CAS Nr. 2842-37-7], 2-[dodecyl(2-hydroxyethyl)amino]ethanol [CAS Nr. 1541-67-9], poly(dimethylamine-co-epichlorohydrin) [CAS Nr. 25988-97-0], N,N-dimethyldodecylamine N-oxide [CAS Nr. 61788-90-7], n,n-dimethyltetradecylamine N-oxide [CAS Nr. 3332-27-2 ], dimethyl myristamine [CAS Nr. 112-75-4], 1-propanamine, n,n-dimethyl-3-(trimethoxysilyl)- [CAS Nr. 2530-86-1].

The examples given (can) contain chloride counter ions, but it applies for all corresponding anions such as, and not limited to, bromide, fluoride, iodide, hydroxide, alkyl sulfate, gluconate, carbonate or bicarbonate, or mixtures thereof.

From the above list of examples, preferred compounds are:
tetradecyldimethyl(3-trimethoxysilylpropyl)ammonium chloride [CAS Nr. 41591-87-1], dimethyloctadecyl[3-(trimethoxysilyl)propyl]ammonium chloride [CAS Nr. 27668-52-6], and dimethyloctadecyl(3-(triethoxysilyl)propyl)ammonium chloride [CAS Nr. 62117-57-1], tetradecyltrimethylammonium chloride [CAS Nr. 4574-04-3], didecyl dimethyl ammonium chloride [CAS Nr. 7173-51-5] and N-alkyl-N-benzyl-N,N-dimethylammonium chloride [CAS Nr. 8001-54-1], C8-18-alkydimethylbenzyl ammonium chlorides [CAS Nr. 63449-41-2], myristyltrimethylammonium chloride [CAS Nr. 4574-04-3], stearyltrimethylammonium chloride [CAS Nr. 112-03-08 ], cetrimonium bromide [CAS Nr. 57-09-0 ], Polyquaternium 6 [CAS Nr. 26062-79-3], 1-decanaminium, N-decyl-N-(2-hydroxyethyl)-N-methyl-, propanoate [CAS Nr. 107879-22-1], and N-Alkyl-N-benzyl-N,N-dimethylammonium chloride [CAS Nr. 8001-54-], bis (3-aminopropyl) dodecylamine [CAS Nr. 2372-82-9], bis(3-Aminopropyl)Amine [CAS Nr: 56-18-8], dimethyldodecylamine N-oxide [CAS Nr. 61788-90-7], N,N-dimethyltetradecylamine N-oxide [CAS Nr. 3332-27-2], dimethyl myristamine [CAS Nr. 112-75-4], and diethyldodecylamine [CAS Nr.4271-27-6].

The at least one organic antimicrobially active component (C) is generally present in the polyurethane composition (P) in an amount of from 50 to 50,000 ppm, preferably from 500 to 40,000 ppm, more preferably from 1,000 to 35,000 ppm.

In one embodiment, component (C) is present in an amount of from 250 to 8,000 ppm, preferably 500 to 6,000 ppm, more preferably 625 to 5,000 ppm, based on the total weight of the polyurethane composition (P), and is dimethyl-tetradecyl(3-(trimethoxysilyl)propyl)ammonium chloride. In another embodiment, component (C) is present in an amount of from 250 to 4,000 ppm, preferably 500 to 3,000 ppm, more preferably 625 to 2,500 ppm, based on the total weight of the polyurethane composition (P), and is dimethyloctadecyl(3-(trimethoxysilyl)propyl)ammonium chloride.

In yet another embodiment, component (C) is present in an amount of from 400 to 40,000 ppm, preferably 800 to 32,000 ppm, more preferably 1,200 to 20,000 ppm, based on the total weight of the polyurethane composition (P), and is selected from quaternary ammonium salts having at least two aliphatic C₈-C₃₀ hydrocarbon moieties attached to the nitrogen atom, preferably didecyldimethyl ammonium chloride. In yet another embodiment, component (C) is present in an amount of from 1,500 to 40,000 ppm, preferably 2,000 to 20,000 ppm, more preferably 2,500 to 10,000 ppm, based on the total weight of the polyurethane composition (P), and is selected from quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride.

In yet another embodiment, component (C) is present in an amount of from 2,500 to 25,000 ppm, preferably 4,000 to 12,000 ppm, more preferably 5,000 to 10,000 ppm, based on the total weight of the polyurethane composition (P), and is selected from quaternary ammonium salts having one C₈-C₃₀ hydrocarbon moiety attached to the nitrogen atom, preferably myristyltrimethylammonium chloride. In yet another embodiment, component (C) is present in an amount of from 1,500 to 15,000 ppm, preferably 2,000 to 10,000 ppm, more preferably 2,500 to 8,000 ppm, based on the total weight of the polyurethane composition (P), and is selected from C₈-C₃₀ alkylamine dipropylenediamines, preferably laurylamine dipropylenediamine. In a further embodiment, component (C) is a mixture of any of the above components, wherein the total amount of antimicrobially active component (C) is in a range of from 500 to 8000 ppm, preferably from 1000 to 4000 ppm, based on the total weight of the polyurethane composition (P).

In a preferred embodiment, the polyurethane composition (P) comprises from 1,000 to 12,000 ppm, preferably 2,000 to 10,000 ppm, based on the total weight of the polyurethane composition (P), of EDTA or a salt thereof as complexing agent (B) and from 1,000 to 1,500 ppm, based on the total weight of the polyurethane composition (P), of dimethyloctadecyl(3-(trimethoxysilyl)-propyl)ammonium chloride as organic antimicrobially active component (C).

In another preferred embodiment, the polyurethane composition (P) comprises from 3,000 to 12,000 ppm, preferably from 5,000 to 10,000 ppm, based on the total weight of the polyurethane composition (P), of EDTA or a salt thereof as complexing agent (B) and from 1,000 to 5,000 ppm, preferably from 2,000 to 3,000 ppm, based on the total weight of the polyurethane composition (P), of quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride as organic antimicrobially active component (C).

In yet another preferred embodiment, the polyurethane composition (P) comprises from 1,000 to 12,000 ppm, preferably from 2,000 to 10,000 ppm, based on the total weight of the polyurethane composition (P), of EDTA or a salt thereof as complexing agent (B) and from 8,000 to 25,000 ppm, preferably from 10,000 to 20,000 ppm, based on the total weight of the polyurethane composition (P), of quaternary ammonium salts having one C₈-C₃₀ hydrocarbon moiety attached to the nitrogen atom, preferably myristyltrimethylammonium chloride as organic antimicrobially active component (C).

In another preferred embodiment, the polyurethane composition (P) comprises from 3,000 to 12,000 ppm, preferably from 5,000 to 10,000 ppm, based on the total weight of the polyurethane composition (P), of EDTA or salts thereof as complexing agent (B) and from 1,500 to 15,000 ppm, preferably from 2,500 to 10,000 ppm, based on the total weight of the polyurethane composition (P), of C₈-C₃₀ alkylamine dipropylenediamine, preferably laurylamine dipropylenediamine as organic antimicrobially active component (C).

### Optional polymer (D):

The polyurethane composition (P) may optionally comprise up to 15% by weight, often 1 to 15% by weight, based on the total weight of the polyurethane composition (P), of one or more polymers (D) different from the polyurethane component (A) and, if applicable, the polymeric compounds contained as complexing agent (B) or organic antimicrobial active component (C).

The one or more polymers (D) may comprise any polymer that is compatible with the polyurethane component (A). For example, the one or more polymers (D), if present, may comprise an acrylic polymer, such as poly(acrylic acid) or salts thereof, poly(methacrylic acid) or salts thereof, poly(alkyl acrylate) or poly(alkyl methacrylate).

In one embodiment, the polyurethane composition (P) comprises from 5 to 15% by weight, preferably from 8 to 14% by weight, more preferably from 10 to 13% by weight, based on the total weight of the polyurethane composition (P), of an acrylic viscosity modifier. In another embodiment, the polyurethane composition (B) comprises from 1 to 4% by weight, preferably from 1 to 3% by weight, based on the total weight of the polyurethane composition (P), of an acrylic resin. In yet another embodiment, the polyurethane composition (B) does not comprise a polymer (D).

### Optional additives (E):

The polyurethane composition (P) may optionally comprise up to 5% by weight, based on the total weight of the polyurethane composition (P), of one or more additives (E). Often, 0.1 to 5% by weight are used.

The one or more additives (E), if present, may comprise any additives that are typically used in the production of polyurethane compositions. Additives that may be used according to the present examples are, e.g. described in "A Handbook of Polymer Foams" (David Eaves, 2004, Smithers Rapra publishing) or in "Szycher,s Handbook of Polyurethanes" (Michael Szycher, 2012, 2. Edition, CRC Press). The additives typically find an implementation in actual industrial polyurethane formulations and may be, e.g. flame retardants, light stabilizers, antioxidants, pigments, dyes, catalysts, blowing agents, surfactants, organic solvents, water, foam stabilizing and foam cell opening agents etc.

Preferably, the one or more additives (E), if present, comprise PDMS based surfactants as foam stabilizing and foam cell opening agents and/or amine catalysts and/or metal based catalysts.

Exemplary additives (E) are acetone, carbon dioxide, butane, or any known blowing agents when water is not used for that purpose. Other additives such as flame retardants are generally added to meet customer requirements.

Examples of PDMS based surfactants are polyoxyalkylene siloxanes and silicones [CAS Nr. 87244-72-2]. Other examples comprise siloxanes and silicones, di-methyl, 3-hydroxypropyl methyl, ethoxylated propoxylated , [CAS Nr. 68937-55-3], siloxanes and silicones, di-methyl, 3-hydroxypropyl methyl, ethers with polyethylene-polypropylene glycol mono-methyl ether [CAS Nr. 67762-85-0], Polyoxyalkylene siloxanes and silicones [CAS Nr. 87244-72-2].

Further examples of organosiloxane surfactants are [CAS Nr. 27306-78-1, 125997-17-3, 68937-54-2 67674-67-3, 63148-55-0]. Further foam stabilizing agents non PDMS based are butylene oxide propylene oxide block copolymer [CAS Nr. 27637-03-2, 27517-34-6] alcohols C9-11-branched and linear ethers with ethyloxirane-oxirane polymer mono-methyl ether [CAS Nr. 111163-38-3] 1,2-butylene oxide-ethylene oxide copolymer ether with dodecyl alcohol [CAS Nr. 39454-63-2], allyloxypolyethyleneglycol methyl ether [CAS Nr. 27252-80-8]. An example of amine catalysts is triethylene diamine [CAS Nr. 280-57-9]. Further examples comprise tetramethylethylenediamine [CAS Nr. 110-18-9], 1-[2-(dimethylamino)ethyl]-4-methylpiperazine [CAS Nr. 104-19-8], N,N-dimethylaminocyclohexane [CAS Nr. 98-94-2], or DABCO 1,4-diazabicyclo[2.2.2]octane [CAS Nr. 280-57-9].

Only ternary amines can serve as catalysts as they are not consumed by the reaction with the isocyanate moiety. Examples of metal complex catalysts are stannous octoate [CAS Nr. 301-10-0], dibutyltin dilaurate (DBTDL) [CAS Nr. 77-58-7], dibutyltin diacetate (DBTDA) [CAS Nr. 1067-33-0], dibutyltin bis(dodecyl mercaptide) [CAS Nr. 1185-81-5], other catalysts are bismuth or zirconium based.

In one embodiment, the polyurethane composition (P) comprises from 0.01 to 5% by weight, preferably from 0.1 to 4% by weight, more preferably from 0.5 to 2% by weight, based on the total weight of the polyurethane composition (P), of one or more additives (E), preferably PDMS based surfactants as foam stabilizing and foam cell opening agents and/or amine catalysts and/or metal based catalyst.

In another embodiment, the polyurethane composition (P) does not contain an additive (E).

A further aspect of the present invention is a polyurethane foam or coating comprising the polyurethane composition (P) as described above. For example, the polyurethane foam or coating of the invention may be a polyurethane foam such as a viscoelastic polyurethane foam, a flexible polyurethane foam, a rigid or hard polyurethane foam, or a polyurethane coating such as a polyurethane film, or a polyurethane dispersion film.

Another aspect of the present invention is an article prepared from the polyurethane composition (P) or the foam or coating as described above. For example, the article may be a mattress, pillow, cushion, wound dressing, shoe inlay, upholstery foam or construction foam, preferably a mattress, pillow, cushion or wound dressing.

Although a subsequent impregnation of cured polyurethane foam to reach higher loading level has been proposed elsewhere, this solution to avoid disturbance of the foaming process is very limited and not very satisfactory in terms of distribution from one side and in terms of processing on the other side. It requires at least two additional manufacturing steps (impregnation/soaking and drying).

The liquid product used for the impregnation will tend to settle down causing a gradient of concentration where higher loadings will be found at the bottom of the foam. This approach is limited to open celled foam as far as the liquid may be able to reach evenly the foam surface. By this procedure the distribution of the compounds for a satisfying overall protection is not insured and the compounds are mostly adsorbed and not included in the material.

It has been surprisingly found, that with the combination of the at least one complexing agent (B) and the at least one organic antimicrobially active component (C) as described above, these components can be added to the polyurethane during the process for its preparation.

Mixing together at least one polyol, the at least one complexing agent (B) and the at least one organic antimicrobially active component (C) as described above has been found to significantly enhance the antimicrobial efficiency of the final cured foam system without causing the typical disturbance on the foaming process.

This allows by a regular process the production of a uniformly treated antimicrobial polyurethane material without subsequent addition of antimicrobial substances or without deterioration of the foam properties and foaming behaviour.

One aspect of the invention is therefore a process for preparing the polyurethane composition (P) as described above, comprising the steps:
I) providing at least one polyol having at least two, preferably two or three hydroxyl moieties and at least one polyisocyanate having at least two, preferably two isocyanate moieties;
II) providing at least one complexing agent (B) and at least one organic antimicrobially active component (C) as described above;
III) optionally providing at least one catalyst for promoting the formation of urethane moieties from the hydroxyl groups of the polyol and the isocyanate groups of the polyisocyanate;
IV) optionally providing one or more further additives (E);
V) mixing the at least one polyol, the at least one complexing agent (B) and the at least one organic antimicrobially active component (C) to obtain a mixture (M);
VI) admixing the at least one polyisocyanate to the mixture (M);
VII) allowing the at least one polyol to react with the at least one polyisocyanate in order to obtain the polyurethane composition (P) as described above;
wherein the optional catalyst and/or the optional one or more additives (E), if present, may be added to the mixture (M) before, after or together with the at least one polyisocyanate.

Another aspect of the present invention is the use of a combination of at least one complexing agent (B) and at least one organic antimicrobially active component (C) and optionally one or more additives (E) as described above for preparing an antimicrobial polyurethane composition.

Yet another aspect of the invention is the use of a combination of at least one complexing agent (B) and at least one organic antimicrobially active component (C) and optionally one or more additives (E) as described above for providing antimicrobial properties on a polyurethane composition.

A kit of components for preparing a polyurethane composition or a polyurethane article, comprising at least one polyol having at least two, preferably two or three hydroxyl moieties; at least one polyisocyanate having at least two, preferably two isocyanate moieties; at least one complexing agent (B) as described above; at least one antimicrobially active component (C) as described above; and optionally one or more additives (E) as described above, is also an aspect of the present invention.

The invention is further illustrated by the examples and claims disclosed below.

### Examples

The preparation of various polyurethane foams and coatings, the evaluation of the foam quality, the tests for antimicrobial efficiency as well as some examples and the results are described and discussed in the following:

### Synthesis example 1: Memory foam A1

100 parts by weight of polyether triol (2.5 pcf Revis polyol Blend, Urethane Sciences, W. Berlin, NJ, USA; CAS Nr. 25791-96-2), 0.12 parts by weight of tin catalyst (KOSMOS 5P, Evonik, Richmond VA, USA; CAS Nr. 301-10-0) and the complexing agent (B) and organic antimicrobially active component (C) specified in individual examples below in the respective amounts, were combined to a total weight of 100 grams.

The formulation with the above-mentioned ingredients was mixed vigorously in a mixing unit (Speed mixer DAC 400. FVZ) for 1 min at 1,500 rpm (rounds per minute) followed by 1 min at 2,000 rpm.

The formulation was further additionally mixed up in a dissolver (Mathis MSM, propeller stirrer) for 10 seconds at 1,500 rpm.

Afterwards 45.93 parts by weight of the isocyanate mixture of 4,4,-methylenediphenyl diisocyanate and o-(p-isocyanatobenzyl)phenyl isocyanate (SUPRASEC^{®} 2629, Huntsman Holland BV, NL;) was added and the mixture was stirred vigorously for 20 seconds at 1,500 rpm using a dissolver (Mathis MSM, propeller stirrer). The mixture was poured into a rectangular form (18 cm x 13 cm x 6 cm) and allowed to react and expand. After 24 hours, pieces of 10 mm thickness, 120 mm width, 90 mm height - each identically cut from the middle of the polyurethane foam - were tempered at 120 °C for 48 hours in a ventilated oven (Binder 9110-0216).

### Synthesis example 2: Memory foam A2

100 parts by weight of polyether triol (2.5 pcf Revis polyol Blend, Urethane Sciences, W. Berlin, NJ, USA; CAS Nr. 25791-96-2), 0.12 parts by weight of tin catalyst (KOSMOS 5P, Evonik, Richmond VA, USA; CAS Nr. 301-10-0) and the complexing agent (B) and organic antimicrobially active component (C) in individual examples below in the respective amounts were combined to a total weight of 100 grams. The formulation with the above-mentioned ingredients has been mixed vigorously in a mixing unit (Speed mixer DAC 400. FVZ) for 1 min at 1,500 rpm followed by 1 min at 2,000 rpm.

The formulation was further additionally mixed up in a dissolver (Mathis MSM, propeller stirrer) for 10 seconds at 1,500 rpm. Afterwards 45.93 parts by weight of the isocyanate mixture of 4,4,-methylenediphenyl diisocyanate and o-(p-isocyanatobenzyl)phenyl isocyanate (SUPRASEC^{®} 2629, Huntsman Holland BV, NL) was added and the mixture was stirred vigorously for 20 seconds at 1,500 rpm using a dissolver (Mathis MSM, propeller stirrer).

The mixture was poured into a rectangular form (18 cm x 13 cm x 6 cm) and allowed to react and expand.

After 24 hours, pieces of 10 mm thickness, 120 mm width, 90 mm height - each identically cut from the middle of the polyurethane foam - were tempered at 160 °C for 2 hours in a ventilated oven (Binder 9110-0216).

### Synthesis example 3: Soft foam A3

100 parts by weight of a polyol blend (Neukadur PU SF 50 Komp. A, Suter Kunststoffe AG, Fraubrunnen, CH) and the complexing agent (B) and organic antimicrobially active component (C) specified in individual examples below in the respective amounts were combined to a total weight of 100 grams.

The formulation with the above-mentioned ingredients has been mixed vigorously in a mixing unit (Speed mixer DAC 400. FVZ) for 1 min at 1,500 rpm followed by 1 min at 2,000 rpm. The formulation was further additionally mixed up in a dissolver (Mathis MSM, propeller stirrer) for 10 seconds at 1,500 rpm.

Afterwards 70 parts by weight of the isocyanate (Neukadur PU SF 50 Komp. B, Suter-Kunststoffe AG, Fraubrunnen, CH; mixture of isocyanates) was added and the mixture was stirred vigorously for 20 seconds at 1,500 rpm using a dissolver (Mathis MSM, propeller stirrer).

The mixture was poured into a rectangular form (18 cm x 13 cm x 6 cm) and allowed to react and expand. After 24 hours, pieces of 10 mm thickness, 120 mm width, 90 mm height - each identically cut from the middle of the polyurethane foam - were tempered at 160 °C for 2 hours in a ventilated oven (Binder 9110-0216).

### Synthesis example 4: Rigid foam A4

100 parts by weight of a polyol blend (PUR 701 Suter-Kunststoffe AG, Fraubrunnen, CH; CAS Nr. not available) and the complexing agent (B) and organic antimicrobially active component (C) specified in individual examples below in the respective amounts were combined to a total weight of 100 grams.

The formulation with the above-mentioned ingredients has been mixed vigorously in a mixing unit (Speed mixer DAC 400. FVZ) for 1 min at 1,500 rpm followed by 1 min at 2,000 rpm. The formulation was further additionally mixed up in a dissolver (Mathis MSM, propeller stirrer) for 10 seconds at 1,500 rpm.

Afterwards 120 parts by weight of the isocyanate mixture (Puronate 900/1, Suter Kunststoffe AG, Fraubrunnen, CH; mixture of diphenylmethane diisocyanate (CAS 9016-87-9) and isocyanate diphenylmethane-4,4,-diisocyanate (CAS Nr. 101-68-8) was added and the mixture was stirred vigorously for 20 seconds at 1,500 rpm using a dissolver (Mathis MSM, propeller stirrer).

The mixture was poured into a rectangular form (18 cm x 13 cm x 6 cm) and allowed to react and expand.

After 24h pieces of 10 mm thickness, 120 mm width, 90 mm height - always identically cut in the middle of the polyurethane foam - are used for antimicrobial efficacy testing.

### Synthesis example 5: Polyurethane dispersion coating A5

100 parts by weight of an aqueous polyurethane dispersion Lurapret N5122 fl, (40% w/w, Archroma Management GmbH, Reinach, CH), 12 parts by weight of an acrylic polymer (10% w/w, Borchi^{®} Gel A LA, Borchers Americas, Inc., Franklin, PA, USA; CAS not available) and the complexing agent (B) and organic antimicrobially active component (C) specified in individual examples below in the respective amounts were combined to a total weight of 50 grams.

The formulation with the above-mentioned ingredients was stirred vigorously in a mixing unit (Speed mixer DAC 400. FVZ) for 2 min at 2,000 rpm.

The polymer dispersion was coated (Mathis Lab dryer LTE-T) with a coating knife (clearance of 0.5 mm) on a release paper.

Afterwards the coating was dried and cured at 120 °C for 10 min before anti-microbial performance testing.

### Evaluation of the experiments

The foam quality has been judged by the three following characteristics:
- Behavior during the foam build-up (A1, A2, A3 and A4)
- Optical evaluation of the foam cell structure (A1, A2, A3 and A4)
- Rating of the resilience of the foam sample after compression

Following quality marks were given:

| | |
|---|---|
| 0 | no foam or coating formed |
| + | very bad or collapsed foam; very defective coating |
| ++ | bad foam quality; defective coating |
| +++ | mediocre foam or coating quality |
| ++++ | good foam or coating quality |
| +++++ | comparable quality with reference foam or coating |

### Testing for antimicrobial efficacy

### A) ASTM G21 test method

The test method ASTM G21 presents a well-defined testing procedure for evaluation of antimicrobial performances of a treated article substrate. In order to investigate the antimicrobial performance of foam article and to underline the superior performance of the invention, a strengthened version of ASTM G21 was carried out, denoted below after as "ASTM G21 strengthened".

Instead of the standard growth medium as defined in the standard ASTM G21, the same growth medium enriched with glucose at a concentration of 3 g/l in the spore solution was used. This growth medium simulates soiling conditions that can be encountered during use.

### Description of the method:

The specimen is placed on the surface of a nutrient salt enriched agar. Then the surface is inoculated including the surface of the test specimens with the composite spore suspension and incubated at 28 °C for 28 days.

Test organisms: *Aspergillus niger* - ATCC 9642, *Penicillium funiculosum* - ATCC 11797, *Chaetomium globosum* - ATCC 6205, *Trichoderma virens* - ATCC 9645, *Aureobasidium pullulans* - ATCC 15233

The detailed composition of the nutrient salt enriched agar and procedure are described in ASTM G21. The obtained agar is sterilized at 120 °C for 20 min and poured into petri dishes for testing ASTM G21 strengthened. A nutrient salt solution of the same composition as the nutrient salt agar medium is prepared without the agar added to it. This solution is used as the inoculum medium. This medium is prepared thus that a 10⁶ spores/mL concentration level is achieved.

The test results were visually evaluated for microbial development on the surface of the specimens using the rating from 0 to 4 according the official ASTM G21 method and as indicated below:

| | |
|---|---|
| 4 | at least 60% of the surface is covered with mold fungi |
| 3 | 30 to 60% of the surface is covered with mold fungi |
| 2 | 10 to 30% of the surface is covered with mold fungi |
| 1 | less than 10% of the surface is covered with mold fungi |
| 0 | no fungal growth detected. |

### B) AATCC 30 part III test method

AATCC test method 30 part III trials were performed for some examples to underline the antimicrobial efficiency and importance of the invention.

For AATCC 30 part III, the detailed composition of the agar and procedure are described in AATCC 30 paragraph 21.1. The agar medium is sterilized at 120 °C for 20 min and poured into petri dishes. A mineral salt solution is used as a growth and transfer medium of the spores of the tested organism: *Aspergillus niger -* ATCC 6275.

The spore suspension is placed on the surface of a nutrient salt enriched agar. Then the specimen is placed on the surface of a nutrient salt enriched agar and incubated at 28 °C for 14 days.

The test results are evaluated according to the AATCC test method 30 part III and as indicated below:

| | |
|---|---|
| No growth | specimen fully protected against fungal growth |
| Mic | microscopic growth, insufficient protection |
| Mac | macroscopic growth, insufficient protection |

### C) EN ISO 846 A test method

For polyurethane dispersion (PUD) coatings, a similar testing procedure to EN ISO 846 method A was performed.

For the test, the specimen is placed on the surface of a non-carbonic nutrient salt enriched agar. The agar surface is inoculated, including the surface of the test specimens, with the composite spore suspension and incubated at 28 °C for 28 days. Test specimens with the dimensions 50 mm x 50 mm in a fivefold determination are prepared.

Test reference organisms: *Aspergillus niger-* ATCC 6275, *Penicillium funiculosum* - ATCC 36839, *Chaetomium globosum* - ATCC 6205, *Trichoderma virens -* ATCC 9645, *Paecilomyces varioti* - ATCC 18502
The detailed composition and procedure are described in EN ISO 846.

The test results are visually evaluated as microbial development on the surface of the specimens in the rating 0 (no growth) to 5 (complete growth) according to the EN ISO 846 method and as indicated below:

| | |
|---|---|
| 0 | No fungal growth, excellent resistance |
| 1 | fungi visible with the microscope, sufficient resistance |
| 2-5 | fungi visible to the naked eye, insufficient resistance |

### D) Agar diffusion test SAN BIO 12/94

For PUD coatings, in addition to EN ISO 846 method A, SAN BIO 12/94 test with the organism *Penicillium funiculosum* - ATCC 36839 was performed similar as described in DIN EN ISO 20645.

This method is suitable for treated materials which show diffusion properties. Circular specimens of 25 mm diameter are placed on Potato dextrose enriched agar (39.0 g/l) for *Penicillium funiculosum.* The test samples were incubated for one day in the refrigerator and after that, 7 days at 28 °C in the incubator.

The evaluation is based on the presence or absence of growth of fungi in the contact zone of the nutritive medium under the specimens as well as on the formation of a possible inhibition zone around the specimen as indicated below:

| | |
|---|---|
| No growth (0) | complete protection against fungal growth |
| Weak (w) | at least 95% of sample is free of fungal growth |
| Medium (m) | insufficient protection, less than 95% free of fungi |
| Full (f) | insufficient protection, sample is covered with fungi |

### E) JIS L 1902 test method

For polyurethane memory foam, a similar testing procedure to JIS L 1902 was performed. This test method is applied for the quantitative determination of the antibacterial effectiveness of active substances.

Specimens of 0.4 g of polyurethane memory foam were contaminated with a standard number of bacteria of a given specific microorganism (inoculum, see below). After incubation of 18 hours by 37 °C, the microorganisms on the test material were washed off with a defined amount medium. The number of colony-forming-units (cfu) was determined and expressed logarithmically. From this number the antimicrobial effect can be calculated.

The detailed composition and procedure are described in JIS L 1902 count test method.

### Test reference organism:

*Escherichia coli* ATCC 11229

Evaluation is based on the difference in bacteria count in terms of cfu (colony forming units) between 0 and 18 hours contact with the test material. Germ reduction "Bacteriostatic Activity S" is given as logarithmic germ reduction (logR activity).

### F) JIS Z 2801 test method

For polyurethane dispersion (PUD) coatings, for underlining the antimicrobial efficiency against bacteria, a similar testing procedure to JIS Z 2801 was performed.

This method is designed for quantitative determining the antibacterial efficacy of materials that have been treated with antibacterial active substances.

The specimens (flat, square with a surface measuring 45 mm x 45 mm) were inoculated with a 120 microliter of a germ-containing suspension (inoculum) and covered with a thin sterile plastic film (35 mm x 35 mm). The inoculated specimens were then incubated in a closed system at 37 °C for 24 hours. After incubation at 37 °C the bacteria were transferred into a specified amount of a diluted solution.

The detailed composition and procedure are described in JIS Z 2801 germ count test method.

### Test reference organism:

### Escherichia coli ATCC 8739

Base of assessment is the difference between the counted amounts of colony forming units (cfu) of the specimens at 0 hours and 24 hours of contact time. The germ reduction indicated as "Bacterial Activity R" is given as logarithmic reduction (logR activity).

### Example 1

The study was initiated to investigate the effects of combinations of dimethyl-tetradecyl(3-(trimethoxysilyl)propyl)ammonium chloride and various complexing agent molecules on their polyurethane foam forming behavior as well as their antimicrobial performance according to ASTM G21, strengthened. The polyurethane foam was memory foam A1.

In a first step, the polyurethane foam quality in the presence of the antimicrobial dimethyltetradecyl(3-(trimethoxysilyl)propyl)ammonium chloride (CAS Nr. 41591-87-1), indicated as "(C)" in the tables below, as well as various complexing agents, indicated as "(B)" in the tables below, was tested. Whereas the control polyurethane foams without or with the antimicrobially active agent dimethyl-tetradecyl(3-(trimethoxysilyl)propyl)ammonium chloride had good foam quality, certain complexing agents reduced foam quality or inhibited foaming under the conditions tested (quality indicated as "Q" in the tables below). Some complexing agents did not affect foam quality significantly compared to the control samples. The solvents used, indicated as "(E)" in the tables below, had no influence on foam quality except for water, which indicates its limited use as solvent in polyurethane foaming.

In a second step, the produced memory foams were tested according ASTM G21 strengthened, as described above, indicated as "G21" in tables below.

Most of the prepared polyurethane foam specimens showed similarly insufficient antimicrobial protection as the control samples with and without the antimicrobial agent.

Disodium EDTA dihydrate (CAS Nr. 6381-92-6, Trilon-BD) and EDTA, tetrasodium (CAS Nr. 64-02-8), indicated very good antimicrobial efficiency with excellent foam quality.

The results are shown in Table 1.

**Table 1 Amounts are given in parts by weight (pbw), as introduced in synthesis examples.**

| (B) | | (C) | (E) | | Q | G21 |
|---|---|---|---|---|---|---|
| Substance | Pbw | pbw | Substance | pbw | | |
| Control | | 0 | - | | +++++ | 4 |
| Control | | 0.5 | - | | ++++ | 3 |
| Disodium EDTA dihydrate | 0.2 | 0.5 | Diglyme | 0.8 | ++++ | 0 |
| Tetrasodium EDTA | 0.2 | 0.5 | Diglyme | 0.8 | ++++ | 0 |
| Undecanoic acid | 0.5 | 0.5 | Arcol Polyol 1108 | 0.5 | ++++ | 4 |
| Sodium glycolate | 0.2 | 0.5 | Diglyme | 0.8 | ++++ | 4 |
| DL-Malic acid disodium salt | 0.3 | 0.5 | Triacetin | 0.8 | ++++ | 4 |
| DL-Tartaric acid | 0.2 | 0.5 | Diglyme | 0.8 | + | 4 |
| Sodium hydrogen tartrate | 0.2 | 0.5 | Diglyme | 0.8 | ++++ | 3 |
| Sodium L-lactate | 0.2 | 0.5 | Triacetin | 0.8 | ++++ | 3 |
| Citric acid | 0.2 | 0.5 | Diglyme | 0.8 | + | 4 |
| Monosodium citrate | 0.2 | 0.5 | Diglyme | 0.8 | + | 4 |
| Trisodium N-(1-carboxylato-ethyl)imidodiacetate | 0.4 | 0.5 | Water | 0.6 | +++ | 4 |
| | 0.2 | 0.5 | Diglyme | 0.8 | ++++ | 3 |
| Nitrilotriacetic acid | 0.2 | 0.5 | Diglyme | 0.8 | + | 4 |
| Ethyleneimine | 0.3 | 0.5 | Diglyme | 0.7 | + | 4 |
| - | - | 0.5 | Triacetin | 1 | ++++ | 4 |
| - | - | 0.5 | Arcol Polyol 1108 | 1 | ++++ | 4 |
| - | - | 0.5 | Diglyme | 1 | ++++ | 4 |
| - | - | 0.5 | Water, dH20 | 1 | +++ | - |

### Example 2

Memory foam quality with different quaternary ammonium compounds and alkylamines at various application concentrations was evaluated:
The polyurethane foam was memory foam A1 and the quality was estimated as indicated under the description of the evaluation of the foam and coating quality.

Table 2 shows that the concentration of the used quaternary ammonium compounds as well as amines has an influence in the memory foam quality depending on the antimicrobial substance used. Concentrations of actives with good foam quality were further investigated in the absence or presence of complexing agents. In Table 2, the indicated pbw are from commercial products with various active content (30 to 100%).

**Table 2 Amounts are given in parts by weight (pbw), as introduced in synthesis examples. Control sample was evaluated with +++++. n.t. = not tested.**

| (C) | Quality at given amount of (C) in pbw | | | | | |
|---|---|---|---|---|---|---|
| Substance/Amount: | 0.25 | 0.5 | 1 | 2 | 4 | 8 |
| Dimethyltetradecyl[3-(trimethoxysilyl)propyl]ammonium chloride (50%) | +++++ | +++++ | ++++ | +++ | n.t. | n.t. |
| Dimethyloctadecyl[3-(trimethoxysilyl)propyl]ammonium chloride (50%) | n.t. | +++ | ++ | + | n.t. | n.t. |
| Didecyl dimethyl ammonium chloride (80%) | +++++ | +++++ | +++++ | ++++ | ++++ | ++ |
| C₈-C₁₈-Alkyldimethylbenzyl-ammonium chloride (100%) | +++++ | +++++ | +++++ | ++++ | ++++ | + |
| Myristyltrimethyl ammonium chloride (100%) | +++++ | +++++ | ++++ | ++++ | ++ | n.t. |
| Poly(dimethyldiallylammonium chloride) (100%) | +++++ | +++++ | +++++ | +++++ | +++++ | +++++ |
| Laurylamine dipropylenediamine (100%) | +++++ | ++++ | ++++ | +++ | + | n.t. |
| Poly(dimethylamine-co-epichlorohydrine (60%) | n.t. | n.t. | n.t. | ++ | n.t. | n.t. |
| N,N-Dimethyldodecylamine N-oxide (30%) | n.t. | n.t. | n.t. | 0 | n.t. | n.t. |

### Example 3

This example underlines the beneficial effects of the invention using the combination of dimethyltetradecyl(3-(trimethoxysilyl)propyl)-ammonium chloride (CAS Nr. 41591-87-1);
"(C)" and the complexing agent disodium EDTA dihydrate (CAS Nr. 6381-92-6);
"(B)" at various concentrations. The polyurethane foam was memory foam A2. The cooperative effect of components (B) and (C) is shown in Table 3.

**Table 3 Amounts are given in parts by weight (pbw), as introduced in synthesis examples. Antimicrobial effect measured according to ASTM G21 strengthened, as described above. n.d. = not determined.**

| | pbw (C) | 0 | 0.125 | 0.25 | 0.5 | 1 |
|---|---|---|---|---|---|---|
| pbw (B) | Q | +++++ | ++++ | ++++ | ++++ | +++ |
| 0 | G21 | 4 | 4 | 3 | 0 | n.d. |
| 0.25 | | 3 | 2 | 0 | 0 | n.d. |
| 0.5 | | 3 | 0 | 0 | 0 | n.d. |
| 1 | | 3 | 0 | 0 | 0 | n.d. |

### Example 4

Example 3 was repeated with dimethyloctadecyl(3-(trimethoxysilyl)propyl)-ammonium chloride (CAS Nr. 27668-52-6) instead of dimethyltetradecyl(3-(trimethoxysilyl)propyl)-ammonium chloride. The cooperative effect of components (B) and (C) is shown in Table 4.

**Table 4 Amounts are given in parts by weight (pbw), as introduced in synthesis examples. Antimicrobial effect measured according to ASTM G21 strengthened, as described above. n.d. = not determined.**

| | pbw (C) | 0 | 0.125 | 0.25 | 0.5 | 1 |
|---|---|---|---|---|---|---|
| pbw (B) | Q | +++++ | ++++ | +++ | ++ | + |
| 0 | G21 | 4 | 4 | 3 | n.d | n.d. |
| 0.25 | | 3 | 1 | 0 | n.d | n.d. |
| 0.5 | | 3 | 0 | 1 | n.d | n.d. |
| 1 | | 3 | 0 | 1 | n.d | n.d. |

### Example 5

Example 3 was repeated with didecyldimethyl ammonium chloride (CAS Nr. 7173-51-5) instead of dimethyltetradecyl(3-(trimethoxysilyl)propyl)-ammonium chloride. The cooperative effect of components (B) and (C) is shown in Table 5.

**Table 5. Amounts are given in parts by weight (pbw), as introduced in synthesis examples. Antimicrobial effect measured according to ASTM G21 strengthened, as described above. n.d. = not determined.**

| | pbw (C) | 0 | 0.2 | 0.8 | 1.6 | 3.2 |
|---|---|---|---|---|---|---|
| pbw (B) | Q | +++++ | +++++ | +++++ | ++++ | ++++ |
| 0 | G21 | 4 | 3 | 1 | 0 | 0 |
| 0.25 | | 3 | 0 | 0 | 0 | 0 |
| 0.5 | | 3 | 0 | 0 | 0 | 0 |
| 1 | | 3 | 0 | 0 | 0 | 0 |

### Example 6

Example 3 was repeated with C₈-C₁₈-alkyldimethylbenzyl ammonium chloride (CAS Nr. 63449-41-2) instead of dimethyltetradecyl(3-(trimethoxysilyl)propyl)-ammonium chloride. The cooperative effect of components (B) and (C) is shown in Table 6.

**Table 6. Amounts are given in parts by weight (pbw), as introduced in synthesis examples. Antimicrobial effect measured according to ASTM G21 strengthened, as described above. n.d. = not determined.**

| | pbw (C) | 0 | 0.25 | 0.5 | 1 | 2 |
|---|---|---|---|---|---|---|
| pbw (B) | Q | +++++ | +++++ | +++++ | ++++ | ++++ |
| 0 | G21 | 4 | 3 | 4 | 4 | 0 |
| 0.25 | | 3 | 2 | 0 | 0 | 1 |
| 0.5 | | 3 | 2 | 0 | 0 | 0 |
| 1 | | 3 | 1 | 0 | 0 | 0 |

### Example 7

Example 3 was repeated with myristyltrimethylammonium chloride (CAS Nr. 4574-04-3) instead of dimethyltetradecyl(3-(trimethoxysilyl)propyl)-ammonium chloride. The cooperative effect of components (B) and (C) is shown in Table 7.

**Table 7. Amounts are given in parts by weight (pbw), as introduced in synthesis examples. Antimicrobial effect measured according to ASTM G21 strengthened, as described above. n.d. = not determined.**

| | pbw (C) | 0 | 0.5 | 1 | 2 | 4 |
|---|---|---|---|---|---|---|
| pbw (B) | Q | +++++ | +++++ | ++++ | ++++ | ++ |
| 0 | G21 | 4 | 2 | 0 | 0 | n.d. |
| 0.25 | | 3 | 0 | 0 | 1 | n.d. |
| 0.5 | | 3 | 1 | 0 | 1 | n.d. |
| 1 | | 3 | 0 | 1 | 1 | n.d. |

### Example 8

Example 3 was repeated with laurylamine dipropylenediamine (CAS Nr. 2372-82-9) instead of dimethyltetradecyl(3-(trimethoxysilyl)propyl)-ammonium chloride. The cooperative effect of components (B) and (C) is shown in Table 8.

**Table 8. Amounts are given in parts by weight (pbw), as introduced in synthesis examples. Antimicrobial effect measured according to ASTM G21 strengthened, as described above. n.d. = not determined.**

| | pbw (C) | 0 | 0.5 | 1 | 2 | 4 |
|---|---|---|---|---|---|---|
| pbw (B) | Q | +++++ | ++++ | ++++ | +++ | + |
| 0 | G21 | 4 | 4 | 4 | n.d. | n.d. |
| 0.25 | | 3 | 0 | 1 | n.d. | n.d. |
| 0.5 | | 3 | 0 | 0 | n.d. | n.d. |
| 1 | | 3 | 0 | 0 | n.d. | n.d. |

### Example 9

To underline again the importance of the invention for the protection of polyurethane foam against fungal growth, a further antimicrobial test was performed. Fungal growth has been evaluated according to AATCC test method 30 part III with an exemplary and medically important fungus *Aspergillus,* using the exemplary fungus *Aspergillus nigerATCC* 6275.

The polyurethane foam was memory foam A2, using the combination of dimethyloctadecyl(3-(trimethoxysilyl)propyl)-ammonium chloride (CAS Nr. 27668-52-6); "(C)" and the complexing agent disodium EDTA dihydrate (CAS Nr. 6381-92-6); "(B)" at various concentrations. The cooperative effect of components (B) and (C) is shown in Table 9.

**Table 9. Amounts are given in parts by weight (pbw), as introduced in synthesis examples. Antimicrobial effect measured according to AATCC test method 30 part III, as described above. n.d. = not determined.**

| | pbw (C) | 0 | 0.125 | 0.25 | 0.5 | 1 |
|---|---|---|---|---|---|---|
| pbw (B) | Q | +++++ | ++++ | +++ | ++ | + |
| 0 | AATCC 30 part III (14d) | Mic | Mic | No growth | n.d. | n.d. |
| 0.25 | | Mic | No growth | No growth | n.d. | n.d. |
| 0.5 | | Mic | No growth | No growth | n.d. | n.d. |
| 1 | | Mic | No growth | No growth | n.d. | n.d. |

### Example 10

Example 9 was repeated with didecyldimethyl ammonium chloride (CAS Nr. 7173-51-5) instead of dimethyloctadecyl(3-(trimethoxysilyl)propyl)-ammonium chloride. The cooperative effect of components (B) and (C) is shown in Table 10.

**Table 10. Amounts are given in parts by weight (pbw), as introduced in synthesis examples. Antimicrobial effect measured according to AATCC test method 30 part III as described above. n.d. = not determined.**

| | pbw (C) | 0 | 0.2 | 1.6 | 3.2 | 6.4 |
|---|---|---|---|---|---|---|
| pbw (B) | Q | ++++ | ++++ | ++++ | ++++ | ++ |
| 0 | AATCC 30 part III (14 d) | Mic | Mic | Mic | No growth | n.d. |
| 0.25 | | Mic | No growth | No growth | No growth | n.d. |
| 0.5 | | Mic | No growth | No growth | No growth | n.d. |
| 1 | | Mic | No growth | No growth | No growth | n.d. |

### Example 11

Example 9 was repeated with C₈-C₁₈-alkyldimethylbenzyl ammonium chloride (CAS Nr. 63449-41-2) instead of dimethyloctadecyl(3-(trimethoxysilyl)propyl)-ammonium chloride. The cooperative effect of components (B) and (C)is shown in Table 11.

**Table 11. Amounts are given in parts by weight (pbw), as introduced in synthesis examples. Antimicrobial effect measured according to AATCC test method 30 part III as described above. n.d. = not determined.**

| | pbw (C) | 0 | 0.25 | 0.5 | 1 | 2 | 4 | 8 |
|---|---|---|---|---|---|---|---|---|
| pbw (B) | Q | +++++ | +++++ | +++++ | ++++ | ++++ | ++++ | + |
| 0 | AATCC 30 part III (14 d) | Mic | Mic | Mic | n.d. | n.d. | n.d. | n.d. |
| 0.25 | | Mic | Mic | Mic | n.d. | n.d. | n.d. | n.d. |
| 0.5 | | Mic | Mic | No growth | n.d. | n.d. | n.d. | n.d. |
| 1 | | Mic | No growth | No growth | n.d. | n.d. | n.d. | n.d. |

### Example 12

Example 9 was repeated with myristyltrimethylammonium chloride (CAS Nr. 4574-04-3) instead of dimethyloctadecyl(3-(trimethoxysilyl)propyl)-ammonium chloride. The cooperative effect of components (B) and (C) is shown in Table 12.

**Table 12. Amounts are given in parts by weight (pbw), as introduced in synthesis examples. Antimicrobial effect measured according to AATCC test method 30 part III, as described above. n.d. = not determined.**

| | pbw (C) | 0 | 0.25 | 0.5 | 1 | 2 | 4 |
|---|---|---|---|---|---|---|---|
| pbw (B) | Q | +++++ | +++++ | +++++ | ++++ | ++++ | ++ |
| 0 | AATCC 30 part III (14 d) | Mic | Mic | Mic | Mic | n.d. | |
| 0.25 | | Mic | Mic | Mic | No growth | n.d. | |
| 0.5 | | Mic | Mic | Mic | No growth | n.d. | |
| 1 | | Mic | Mic | Mic | No growth | n.d. | |

### Example 13

Example 9 was repeated with laurylamine dipropylenediamine (CAS Nr. 2372-82-9) instead of dimethyloctadecyl(3-(trimethoxysilyl)propyl)-ammonium chloride. The cooperative effect of components (B) and (C) is shown in Table 13.

**Table 13. Amounts are given in parts by weight (pbw), as introduced in synthesis examples. Antimicrobial effect measured according to AATCC test method 30 part III, as described above. n.d. = not determined.**

| | pbw (C) | 0 | 0.25 | 0.5 | 1 | 2 | 4 |
|---|---|---|---|---|---|---|---|
| pbw (B) | Q | +++++ | ++++ | ++++ | ++++ | +++ | + |
| 0 | AATCC 30 part III (14 d) | Mic | Mic | Mic | Mic | n.d. | n.d. |
| 0.25 | | Mic | Mic | Mic | Mic | n.d. | n.d. |
| 0.5 | | Mic | No growth | No growth | No growth | n.d. | n.d. |
| 1 | | Mic | No growth | No growth | No growth | n.d. | n.d. |

### Example 14

The antimicrobial compound C₈-C₁₈-alkyldimethylbenzyl ammonium chloride (CAS Nr. 63449-41-2), "(C)" and different complexing agents "(B)" were used as additives in memory foam A2 and the anti-microbial effect was estimated according to ASTM G21 strengthened and AATCC test method 30 part III, as described above. In each case with 0.5 pbw of (B) and 0.25 pbw of (C) were used. The quality of the foam was not affected in each case. The results are shown in Table 14.

**Table 14**

| | 0 pbw of (C) | | 0.25 pbw of (C) | |
|---|---|---|---|---|
| (B) (0.5 pbw) | ASTM G21 | AATCC 30 part III | ASTM G21 | AATCC 30 part III |
| Control | 4 | Mic | 2 | Mic |
| Diethylenetriamine pentaacetic acid | 2 | Mic | 3 | Mic |
| N-(2-Hydroxyethyl)-ethylenediamine- | 3 | Mic | 2 | Mic |
| N,N',N'-triacetic acid | | | | |
| Disodium EDTA dihydrate | 3 | Mic | 0 | No growth |

### Example 15

Rigid polyurethane foam A4 was prepared with and without C₈-C₁₈-alkyldimethylbenzyl ammonium chloride as (C) and with and without disodium EDTA dihydrate as (B). The quality of the rigid foam was not affected by the additives. The antimicrobial effect was evaluated according to ASTM G21 strengthened as described above. The cooperative effect of components (B) and (C) is shown in Table 15.

**Table 15**

| | 0 pbw of (C) | 0.25 pbw of (C) |
|---|---|---|
| 0 pbw of (B) | 4 | 3 |
| 0.5 pbw of (B) | 4 | 0 |

### Example 16

Soft polyurethane foam A3 was prepared with varying amounts of dimethyl-tetradecyl(3-(trimethoxysilyl)propyl)ammonium chloride as (C) and with varying amounts of disodium EDTA dihydrate as (B). The quality of the soft foam was not affected by the additives. The antimicrobial effect was evaluated according to AATCC 30, part III as described above. The cooperative effect of components (B) and (C) is shown in Table 16.

**Table 16**

| | 0 pbw of (C) | 0.125 pbw of (C) | 0.5 pbw of (C) |
|---|---|---|---|
| 0 pbw of (B) | Mic | Mic | Mic |
| 0.5 pbw of (B) | Mic | Mic | No growth |
| 1 pbw of (B) | Mic | No growth | No growth |

### Example 17

Polyurethane coating A5 was prepared with varying amounts of dimethyl-tetradecyl(3-(trimethoxysilyl)propyl)ammonium chloride as (C) and with varying amounts of the complexing agent tetrasodium EDTA salt as (B). The quality of the coating was not affected by the additives. The antimicrobial effect was evaluated according to EN ISO 846 A test method as described above. The cooperative effect of components (B) and (C) is shown in Table 17.

**Table 17**

| | 0 pbw of (C) | 0.5 pbw of (C) |
|---|---|---|
| 0 pbw of (B) | 5 | 5 |
| 0.25 pbw of (B) | 5 | 3 |
| 0.5 pbw of (B) | 5 | 1 |
| 1 pbw of (B) | 5 | 0 |

### Example 18

Polyurethane coating A5 was prepared with varying amounts of dimethyl-tetradecyl(3-(trimethoxysilyl)propyl)ammonium chloride as (C) and with varying amounts of disodium EDTA dihydrate as (B). The quality of the coating was not affected by the additives. The antimicrobial effect was evaluated according to SAN BIO 12-94 as described above with *Penicillium funiculosum.* The cooperative effect of components (B) and (C) is shown in Table 18.

**Table 18**

| | 0 pbw of (C) | 0.5 pbw of (C) |
|---|---|---|
| 0 pbw of (B) | Full (f) | Full (f) |
| 0.25 pbw of (B) | Full (f) | No growth (0) |
| 0.5 pbw of (B) | Full (f) | No growth (0) |
| 1 pbw of (B) | Full (f) | No growth (0) |

### Example 19

This example underlines the beneficial effects of the invention using the combination of dimethyltetradecyl(3-(trimethoxysilyl)propyl)-ammonium chloride (CAS Nr. 41591-87-1); "(C)" and the complexing agent disodium EDTA dihydrate (CAS Nr. 6381-92-6); "(B)" at various concentrations against bacteria. Whereas the untreated foam indicates strong bacterial growth, in the combination foam absence of bacterial growth was detected. The polyurethane foam was memory foam A2. The cooperative effect of components (B) and (C) is shown in Table 19.

**Table 19 Amounts are given in parts by weight (pbw), as introduced in synthesis examples.**

| Antimicrobial effect measured according to JIS L 1902 and *Escherichia coli,* as described above. The results indicate logarithmic reduction of bacterial growth (logR). | | |
|---|---|---|
| | 0 pbw (C) | 1 pbw (C) |
| 0 pbw (B) | 0.7 | 2.7 |
| 0.25 pbw (B) | 3.1 | >5.5 |

### Example 20

Polyurethane coating A5 was prepared with varying amounts of dimethyl-tetradecyl(3-(trimethoxysilyl)propyl)ammonium chloride as (C) and with varying amounts of the complexing agent tetrasodium EDTA salt as (B). The quality of the coating was not affected by the additives. The antimicrobial effect was evaluated according to JIS Z 2801 as described above with *Escherichia coli.* The cooperative effect of components (B) and (C) is shown in Table 20.

**Table 20**

| | 0 pbw of (C) | 0.125 pbw of (C) | 0.5 pbw of (C) |
|---|---|---|---|
| 0 pbw of (B) | 0.20 | 0.10 | 0.50 |
| 0.25 pbw of (B) | 0.90 | 2.80 | 2.90 |
| 0.5 pbw of (B) | 3.20 | >5.10 | >5.10 |

## Claims

1. A polyurethane composition (P) comprising:
a) from 80 to 99.9% by weight, based on the total weight of the polyurethane composition (P), of at least one polyurethane component (A);
b) from 0.05 to 1.5% by weight (500 to 15,000 ppm), based on the total weight of the polyurethane composition (P), of at least one complexing agent (B);
c) from 0.005 to 5% by weight (50 to 50,000 ppm), based on the total weight of the polyurethane composition (P), of at least one organic antimicrobially active component (C) comprising at least one nitrogen atom;
d) optionally up to 15% by weight, based on the total weight of the polyurethane composition (P), of one or more polymers (D) different from the at least one polyurethane component (A) and
e) optionally up to 5% by weight, based on the total weight of the polyurethane composition (P), of one or more additives (E);
wherein the at least one complexing agent (B) is selected from the group consisting of organic compounds having at least two functional moieties, of which at least one functional moiety is a carboxylic acid moiety or a salt thereof; and
wherein the at least one organic antimicrobially active component (C) comprising at least one nitrogen atom is selected from the group consisting of quaternary ammonium salts having at least one C₈-C₃₀ hydrocarbon moiety attached to the nitrogen atom, primary, secondary or tertiary amines having at least one C₈-C₃₀ hydrocarbon moiety attached to the nitrogen atom, N-oxides of tertiary amines having at least one C₈-C₃₀ hydrocarbon moiety attached to the nitrogen atom, tertiary amines having a C₈-C₃₀ hydrocarbon moiety and two aminoalkyl moieties attached to the tertiary nitrogen atom, and polymers having repeating units comprising quaternary ammonium moieties or amine moieties.

2. The polyurethane composition (P) according to claim 1, wherein the at least one complexing agent (B) is selected from the group consisting of ethylenediamine tetraacetic acid (EDTA) or salts thereof, glycolic acid or salts thereof, malic acid or salts thereof, tartaric acid or salts thereof, lactic acid or salts thereof, citric acid or salts thereof, N-(1-carboxylato-ethyl)-iminodiacetic acid or salts thereof, nitrilotriacetic acid or salts thereof.

3. The polyurethane composition (P) according to claim 1 or 2, wherein the at least one complexing agent (B) is selected from ethylenediamine tetraacetic acid (EDTA) or salts thereof, preferably the disodium salt thereof or the tetrasodium salt thereof.

4. The polyurethane composition (P) according to any one of claims 1 to 3, wherein the at least one antimicrobially active component (C) is selected from the group consisting of quaternary ammonium salts having at least one aliphatic C₈-C₃₀ hydrocarbon moiety and at least one alkoxylated silylalkyl moiety attached to the nitrogen atom, N-oxides of tertiary amines having at least one C₈-C₃₀ hydrocarbon moiety attached to the nitrogen atom, quaternary ammonium salts having at least two aliphatic C₈-C₃₀ hydrocarbon moieties attached to the nitrogen atom, quaternary ammonium salts having at least one aliphatic C₈-C₃₀ hydrocarbon moiety and at least one aromatic C₆-C₁₄ hydrocarbon moiety attached to the nitrogen atom, poly(diallyldimethyl-ammonium chloride), poly(dialkylamine-co-epichlorohydrine), C₈-C₃₀ alkylamine dipropylenediamine and polyethylene imine).

5. The polyurethane composition (P) according to any one of claims 1 to 4, wherein the at least one antimicrobially active component (C) is selected from quaternary ammonium salts having at least one aliphatic C₈-C₃₀ hydrocarbon moiety and at least one alkoxylated silylalkyl moiety attached to the nitrogen atom, preferably C₈-C₃₀-alkyldimethyl(3-trialkoxysilyl)propyl ammonium salts, more preferably dimethyltetradecyl(3-(trimethoxysilyl)propyl)ammonium chloride or dimethyloctadecyl(3-(trimethoxysilyl)propyl)ammonium chloride.

6. The polyurethane composition (P) according to any one of claims 1 to 5, wherein the at least one complexing agent (B) is present in an amount of from 1,000 to 12,000 ppm, preferably in an amount of from 1,500 to 10,000 ppm, more preferably in an amount of from 2,000 to 8,000 ppm, based on the total weight of the polyurethane composition (P).

7. The polyurethane composition (P) according to any one of claims 1 to 6, wherein the at least one organic antimicrobially active component (C) comprising at least one nitrogen atom, is present in amounts of:
i) 250 to 8,000 ppm, preferably 500 to 6,000 ppm, more preferably 625 to 5,000 ppm, based on the total weight of the polyurethane composition (P), and is dimethyltetradecyl(3-(trimethoxysilyl)propyl)ammonium chloride;
ii) 250 to 4,000 ppm, preferably 500 to 3,000 ppm, more preferably 625 to 2,500 ppm, based on the total weight of the polyurethane composition (P), and is dimethyloctadecyl(3-(trimethoxysilyl)propyl)ammonium chloride;
iii) 400 to 40,000 ppm, preferably 600 to 32,000 ppm, more preferably 800 to 12,000 ppm, even more preferably 1000 to 8,000 ppm, based on the total weight of the polyurethane composition (P), and is selected from quaternary ammonium salts having at least two aliphatic C₈-C₃₀ hydrocarbon moieties attached to the nitrogen atom, preferably didecyldimethyl ammonium chloride;
iv) 1,500 to 10,000 ppm, preferably 2,000 to 8,000 ppm, more preferably 2,500 to 5,000 ppm, based on the total weight of the polyurethane composition (P), and is selected from quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride;
v) 2,500 to 25,000 ppm, preferably 4,000 to 12,000 ppm, more preferably 5,000 to 10,000 ppm, based on the total weight of the polyurethane composition (P), and is selected from quaternary ammonium salts having one C₈-C₃₀ hydrocarbon moiety attached to the nitrogen atom, preferably myristyltrimethylammonium chloride; or
vi) 1,500 to 15,000 ppm, preferably 2,000 to 10,000 ppm, more preferably 2,500 to 8,000 ppm, based on the total weight of the polyurethane composition (P), and is selected from C₈-C₃₀ alkylamine dipropylenediamines, preferably laurylamine dipropylenediamine.

8. The polyurethane composition (P) according to any one of claims 1 to 6, comprising:
i) 1,000 to 12,000 ppm, preferably 2,000 to 10,000 ppm, based on the total weight of the polyurethane composition (P), of EDTA or a salt thereof as complexing agent (B) and 1,000 to 1,500 ppm, based on the total weight of the polyurethane composition (P), of dimethyloctadecyl(3-(trimethoxysilyl)-propyl)ammonium chloride as organic antimicrobially active component (C);
ii) 3,000 to 12,000 ppm, preferably 5,000 to 10,000 ppm, based on the total weight of the polyurethane composition (P), of EDTA or a salt thereof as complexing agent (B) and 1,000 to 6,000 ppm, preferably 2,000 to 5000 ppm, based on the total weight of the polyurethane composition (P), of quaternary ammonium salts having at least one aliphatic C₈-C₃₀ hydrocarbon moiety and at least one aromatic C₆-C₁₄ hydrocarbon moiety attached to the nitrogen atom, preferably C₈-C₁₈-alkyldimethylbenzyl ammonium chloride as organic antimicrobially active component (C);
iii) 1,000 to 12,000 ppm, preferably 2,000 to 10,000 ppm, based on the total weight of the polyurethane composition (P), of EDTA or a salt thereof as complexing agent (B) and 8,000 to 25,000 ppm, preferably 10,000 to 20,000 ppm, based on the total weight of the polyurethane composition (P), of quaternary ammonium salts having one C₈-C₃₀ hydrocarbon moiety attached to the nitrogen atom, preferably myristyltrimethylammonium chloride as organic antimicrobially active component (C); or
iv) 3,000 to 12,000 ppm, preferably 5,000 to 10,000 ppm, based on the total weight of the polyurethane composition (P), of EDTA or salts thereof as complexing agent (B) and 1,500 to 15,000 ppm, preferably 2,500 to 10,000 ppm, based on the total weight of the polyurethane composition (P), of C₈-C₃₀ alkylamine dipropylenediamine, preferably laurylamine dipropylenediamine as organic antimicrobially active component (C).

9. A polyurethane foam or coating comprising the polyurethane composition (P) according to any one of claims 1 to 8.

10. An article, preferably a mattress, pillow, cushion or wound dressing, prepared from the polyurethane composition (P) according to any one of claims 1 to 8 or the foam or coating according to claim 9.

11. A process for preparing the polyurethane composition (P) according to any one of claims 1 to 8 comprising the steps:
I) providing at least one polyol having at least two, preferably two or three hydroxyl moieties and at least one polyisocyanate having at least two, preferably two isocyanate moieties;
II) providing at least one complexing agent (B) and at least one organic antimicrobially active component (C) as described in any one of claims 1 to 8;
III) optionally providing at least one catalyst for promoting the formation of urethane moieties from the hydroxyl groups of the polyol and the isocyanate groups of the polyisocyanate;
IV) optionally providing one or more additives (E);
V) mixing the at least one polyol, the at least one complexing agent (B) and the at least one organic antimicrobially active component (C) to obtain a mixture (M);
VI) admixing the at least one polyisocyanate to the mixture (M);
VII) allowing the at least one polyol to react with the at least one polyisocyanate in order to obtain the polyurethane composition (P) according to any of claims 1 to 8;
wherein the optional catalyst and/or the optional one or more additives (E), if present, may be added to the mixture (M) before, after or together with the at least one polyisocyanate.

12. The use of a combination of at least one complexing agent (B) and at least one organic antimicrobially active component (C) and optionally one or more additives (E) as described in any one of claims 1 to 8 for preparing an antimicrobial polyurethane composition.

13. The use of a combination of at least one complexing agent (B) and at least one organic antimicrobially active component (C) and optionally one or more additives (E) as described in any one of claims 1 to 8 for providing antimicrobial properties on a polyurethane composition.

14. A kit of components for preparing a polyurethane composition or a polyurethane article, comprising at least one polyol having at least two, preferably two or three hydroxyl moieties; at least one polyisocyanate having at least two, preferably two isocyanate moieties; at least one complexing agent (B) as described in any one of claims 1 to 8; at least one antimicrobially active component (C) as described in any one of claims 1 to 8; and optionally one or more additives (E) as described in any one of claims 1 to 8.

## Patentansprüche

1. Polyurethanzusammensetzung (P), umfassend:
a) 80 bis 99,9 Gew.-%, bezogen auf das Gesamtgewicht der Polyurethanzusammensetzung (P), mindestens einer Polyurethankomponente (A);
b) 0,05 bis 1,5 Gew.-% (500 bis 15.000 ppm), bezogen auf das Gesamtgewicht der Polyurethanzusammensetzung (P), mindestens eines Komplexbildners (B);
c) 0,005 bis 5 Gew.-% (50 bis 50.000 ppm), bezogen auf das Gesamtgewicht der Polyurethanzusammensetzung (P), mindestens einer organischen antimikrobiell wirksamen Komponente (C), die mindestens ein Stickstoffatom enthält;
d) optional bis zu 15 Gew.-%, bezogen auf das Gesamtgewicht der Polyurethanzusammensetzung (P), eines oder mehrerer Polymere (D), die sich von der mindestens einen Polyurethankomponente (A) unterscheiden, und
e) optional bis zu 5 Gew.-%, bezogen auf das Gesamtgewicht der Polyurethanzusammensetzung (P), eines oder mehrerer Additive (E);
wobei der mindestens eine Komplexbildner (B) ausgewählt ist aus der Gruppe bestehend aus organischen Verbindungen mit mindestens zwei funktionellen Gruppen, von denen mindestens eine funktionelle Gruppe ein Carbonsäuregruppe oder ein Salz davon ist; und
wobei die mindestens eine organische antimikrobiell wirksame Komponente (C), die mindestens ein Stickstoffatom umfasst, aus der Gruppe ausgewählt ist, die aus quaternären Ammoniumsalzen mit mindestens einer an das Stickstoffatom gebundenen C₈-C₃₀-Kohlenwasserstoffgruppe, primären, sekundären oder tertiären Aminen mit mindestens einer an das Stickstoffatom gebundenen C₈-C₃₀-Kohlenwasserstoffgruppe, N-Oxiden von tertiären Aminen mit mindestens einer an das Stickstoffatom gebundenen C₈-C₃₀-Kohlenwasserstoffgruppe, tertiären Aminen mit einer C₈-C₃₀-Kohlenwasserstoffgruppe und zwei Aminoalkylgruppen, die an das tertiäre Stickstoffatom gebunden sind, und Polymeren mit sich wiederholenden Einheiten, die quaternäre Ammoniumgruppen oder Amingruppen umfassen, besteht.

2. Polyurethanzusammensetzung (P) gemäß Anspruch 1, wobei der mindestens eine Komplexbildner (B) ausgewählt ist aus der Gruppe bestehend aus Ethylendiamintetraessigsäure (EDTA) oder deren Salzen, Glykolsäure oder deren Salzen, Äpfelsäure oder deren Salzen, Weinsäure oder deren Salzen, Milchsäure oder deren Salzen, Zitronensäure oder deren Salzen, N-(1-Carboxylato-ethyl)-iminodiessigsäure oder deren Salzen, Nitrilotriessigsäure oder deren Salzen.

3. Polyurethanzusammensetzung (P) gemäß Anspruch 1 oder 2, wobei der mindestens eine Komplexbildner (B) ausgewählt ist aus Ethylendiamintetraessigsäure (EDTA) oder Salzen davon, bevorzugt dem Dinatriumsalz davon oder dem Tetranatriumsalz davon.

4. Polyurethanzusammensetzung (P) gemäß einem beliebigen der Ansprüche 1 bis 3, wobei die mindestens eine antimikrobiell wirksame Komponente (C) ausgewählt ist aus der Gruppe bestehend aus quaternären Ammoniumsalzen mit mindestens einer aliphatischen C₈-C₃₀-Kohlenwasserstoffgruppe und mindestens einer an das Stickstoffatom gebundenen alkoxylierten Silylalkylgruppe, N-Oxiden von tertiären Aminen mit mindestens einer an das Stickstoffatom gebundenen C₈-C₃₀-Kohlenwasserstoffgruppe, quaternäre Ammoniumsalze mit mindestens zwei an das Stickstoffatom gebundenen aliphatischen C₈-C₃₀-Kohlenwasserstoffgruppen, quaternäre Ammoniumsalze mit mindestens einer aliphatischen C₈-C₃₀-Kohlenwasserstoffgruppe und mindestens einer aromatischen C₆-C₁₄-Kohlenwasserstoffgruppe, die an das tertiäre Stickstoffatom gebunden sind, Poly(diallyldimethylammoniumchlorid), Poly(dialkylamin-co-epichlorhydrin), C₈-C₃₀-Alkylamin-dipropylendiamin und Poly(ethylenimin).

5. Polyurethanzusammensetzung (P) gemäß einem der Ansprüche 1 bis 4, wobei die mindestens eine antimikrobiell wirksame Komponente (C) ausgewählt ist aus quaternären Ammoniumsalzen mit mindestens einer aliphatischen C₈-C₃₀-Kohlenwasserstoffgruppe und mindestens einer alkoxylierten Silylalkylgruppe, die an das Stickstoffatom gebunden sind, bevorzugt C₈-C₃₀-Alkyldimethyl(3-trialkoxysilyl)propylammoniumsalze, mehr bevorzugt Dimethyltetradecyl(3-(trimethoxysilyl)propyl)ammoniumchlorid oder Dimethyloctadecyl(3-(trimethoxysilyl)propyl)ammoniumchlorid.

6. Polyurethanzusammensetzung (P) gemäß einem beliebigen der Ansprüche 1 bis 5, wobei der mindestens eine Komplexbildner (B) in einer Menge von 1.000 bis 12.000 ppm, bevorzugt in einer Menge von 1.500 bis 10.000 ppm, mehr bevorzugt in einer Menge von 2.000 bis 8.000 ppm, bezogen auf das Gesamtgewicht der Polyurethanzusammensetzung (P), vorliegt.

7. Polyurethanzusammensetzung (P) gemäß einem beliebigen der Ansprüche 1 bis 6, wobei die mindestens eine organische antimikrobiell wirksame Komponente (C), die mindestens ein Stickstoffatom umfasst, vorhanden ist in Mengen von:
i) 250 bis 8.000 ppm, bevorzugt 500 bis 6.000 ppm, mehr bevorzugt 625 bis 5.000 ppm, bezogen auf das Gesamtgewicht der Polyurethanzusammensetzung (P), und Dimethyltetradecyl(3-(trimethoxysilyl)propyl)ammoniumchlorid ist;
ii) 250 bis 4.000 ppm, bevorzugt 500 bis 3.000 ppm, mehr bevorzugt 625 bis 2.500 ppm, bezogen auf das Gesamtgewicht der Polyurethanzusammensetzung (P), und Dimethyloctadecyl(3-(trimethoxysilyl)propyl)ammoniumchlorid ist;
iii) 400 bis 40.000 ppm, bevorzugt 600 bis 32.000 ppm, mehr bevorzugt 800 bis 12.000 ppm, noch mehr bevorzugt 1.000 bis 8.000 ppm, bezogen auf das Gesamtgewicht der Polyurethanzusammensetzung (P), und ausgewählt ist aus quaternären Ammoniumsalzen mit mindestens zwei an das Stickstoffatom gebundenen aliphatischen C₈-C₃₀-Kohlenwasserstoffgruppen, bevorzugt Didecyldimethylammoniumchlorid;
iv) 1.500 bis 10.000 ppm, bevorzugt 2.000 bis 8.000 ppm, mehr bevorzugt 2.500 bis 5.000 ppm, bezogen auf das Gesamtgewicht der Polyurethanzusammensetzung (P), und ausgewählt ist aus quaternären Ammoniumsalzen mit mindestens einer aliphatischen C₈-C₃₀-Kohlenwasserstoffgruppe und mindestens einer aromatischen C₆-C₁₄-Kohlenwasserstoffgruppe, die an das Stickstoffatom gebunden sind, bevorzugt C₈-C₁₈-Alkyldimethylbenzylammoniumchlorid;
v) 2.500 bis 25.000 ppm, bevorzugt 4.000 bis 12.000 ppm, besonders bevorzugt 5.000 bis 10.000 ppm, bezogen auf das Gesamtgewicht der Polyurethanzusammensetzung (P), und ausgewählt ist aus quaternären Ammoniumsalzen mit einer an das Stickstoffatom gebundenen C₈-C₃₀-Kohlenwasserstoffgruppe, bevorzugt Myristyltrimethylammoniumchlorid; oder
vi) 1.500 bis 15.000 ppm, bevorzugt 2.000 bis 10.000 ppm, mehr bevorzugt 2.500 bis 8.000 ppm, bezogen auf das Gesamtgewicht der Polyurethanzusammensetzung (P), und ausgewählt ist aus C₈-C₃₀-Alkylamin-Dipropylendiaminen, bevorzugt Laurylamin-Dipropylendiamin.

8. Polyurethanzusammensetzung (P) gemäß einem beliebigen der Ansprüche 1 bis 6, umfassend:
i) 1.000 bis 12.000 ppm, bevorzugt 2.000 bis 10.000 ppm, bezogen auf das Gesamtgewicht der Polyurethanzusammensetzung (P), EDTA oder ein Salz davon als Komplexbildner (B) und 1.000 bis 1.500 ppm, bezogen auf das Gesamtgewicht der Polyurethanzusammensetzung (P), Dimethyloctadecyl(3-(trimethoxysilyl)-propyl)ammoniumchlorid als organische antimikrobiell wirksame Komponente (C);
ii) 3.000 bis 12.000 ppm, bevorzugt 5.000 bis 10.000 ppm, bezogen auf das Gesamtgewicht der Polyurethanzusammensetzung (P), von EDTA oder einem Salz davon als Komplexbildner (B) und 1.000 bis 6.000 ppm, bevorzugt 2.000 bis 5.000 ppm, bezogen auf das Gesamtgewicht der Polyurethanzusammensetzung (P), von quaternären Ammoniumsalzen mit mindestens einer aliphatischen C₈-C₃₀-Kohlenwasserstoffgruppe und mindestens einer aromatischen C₆-C₁₄-Kohlenwasserstoffgruppe, die an das Stickstoffatom gebunden sind, bevorzugt C₈-C,₈-Alkyldimethylbenzylammoniumchlorid als organische antimikrobiell wirksame Komponente (C);
iii) 1.000 bis 12.000 ppm, bevorzugt 2.000 bis 10.000 ppm, bezogen auf das Gesamtgewicht der Polyurethanzusammensetzung (P), von EDTA oder einem Salz davon als Komplexbildner (B) und 8.000 bis 25.000 ppm, bevorzugt 10.000 bis 20.000 ppm, bezogen auf das Gesamtgewicht der Polyurethanzusammensetzung (P), quaternäre Ammoniumsalze mit einer an das Stickstoffatom gebundenen C₈-C₃₀-Kohlenwasserstoffgruppe, bevorzugt Myristyltrimethylammoniumchlorid, als organische antimikrobiell wirksame Komponente (C); oder
iv) 3.000 bis 12.000 ppm, bevorzugt 5.000 bis 10.000 ppm, bezogen auf das Gesamtgewicht der Polyurethanzusammensetzung (P), EDTA oder Salze davon als Komplexbildner (B) und 1.500 bis 15.000 ppm, bevorzugt 2.500 bis 10.000 ppm, bezogen auf das Gesamtgewicht der Polyurethanzusammensetzung (P), C₈-C₃₀-Alkylamindipropylendiamin, bevorzugt Laurylamindipropylendiamin, als organische antimikrobiell wirksame Komponente (C).

9. Polyurethanschaum oder -beschichtung, umfassend die Polyurethanzusammensetzung (P) gemäß einem beliebigen der Ansprüche 1 bis 8.

10. Gegenstand, bevorzugt eine Matratze, ein Kissen, ein Polster oder ein Wundverband, hergestellt aus der Polyurethanzusammensetzung (P) gemäß einem beliebigen der Ansprüche 1 bis 8 oder dem Schaumstoff oder der Beschichtung gemäß Anspruch 9.

11. Verfahren zur Herstellung der Polyurethanzusammensetzung (P) gemäß einem beliebigen der Ansprüche 1 bis 8, umfassend die Schritte:
I) Bereitstellung mindestens eines Polyols mit mindestens zwei, bevorzugt zwei oder drei Hydroxylgruppen und mindestens eines Polyisocyanats mit mindestens zwei, bevorzugt zwei Isocyanatgruppen;
II) Bereitstellen mindestens eines Komplexbildners (B) und mindestens einer organischen antimikrobiell wirksamen Komponente (C) wie in einem beliebigen der Ansprüche 1 bis 8 beschrieben;
III) optional Bereitstellung mindestens eines Katalysators zur Förderung der Bildung von Urethanangruppen aus den Hydroxylgruppen des Polyols und den Isocyanatgruppen des Polyisocyanats;
IV) optionales Bereitstellen eines oder mehrerer Additive (E);
V) Mischen des mindestens einen Polyols, des mindestens einen Komplexbildners (B) und der mindestens einen organischen antimikrobiell wirksamen Komponente (C), um ein Gemisch (M) zu erhalten;
VI) Zumischen des mindestens einen Polyisocyanats zu dem Gemisch (M);
VII) Reagierenlassen des mindestens einen Polyols mit dem mindestens einen Polyisocyanat, um die Polyurethanzusammensetzung (P) gemäß einem beliebigen der Ansprüche 1 bis 8 zu erhalten;
wobei der optionale Katalysator und/oder das optionale eine oder mehrere Additiv(e) (E), falls vorhanden, dem Gemisch (M) vor, nach oder zusammen mit dem mindestens einen Polyisocyanat zugesetzt werden können.

12. Verwendung einer Kombination aus mindestens einem Komplexbildner (B) und mindestens einer organischen antimikrobiell wirksamen Komponente (C) und optional einem oder mehreren Additiven (E), wie in einem beliebigen der Ansprüche 1 bis 8 beschrieben, zur Herstellung einer antimikrobiellen Polyurethanzusammensetzung.

13. Verwendung einer Kombination aus mindestens einem Komplexbildner (B) und mindestens einer organischen antimikrobiell wirksamen Komponente (C) und optional einem oder mehreren Additiven (E), wie in einem beliebigen der Ansprüche 1 bis 8 beschrieben, zur Bereitstellung antimikrobieller Eigenschaften auf einer Polyurethanzusammensetzung.

14. Satz von Komponenten zur Herstellung einer Polyurethanzusammensetzung oder eines Polyurethanartikels, umfassend mindestens ein Polyol mit mindestens zwei, bevorzugt zwei oder drei Hydroxylgruppen; mindestens ein Polyisocyanat mit mindestens zwei, bevorzugt zwei Isocyanatgruppen; mindestens einen Komplexbildner (B), wie in einem beliebigen der Ansprüche 1 bis 8 beschrieben; mindestens eine antimikrobiell wirksame Komponente (C), wie in einem beliebigen der Ansprüche 1 bis 8 beschrieben; und optional ein oder mehrere Zusatzstoffe (E), wie in einem beliebigen der Ansprüche 1 bis 8 beschrieben.

## Revendications

1. Composition de polyuréthane (P) comprenant :
a) de 80 à 99,9 % en poids, sur la base du poids total de la composition de polyuréthane (P), d'au moins un composant polyuréthane (A) ;
b) de 0,05 à 1,5 % en poids (500 à 15 000 ppm), sur la base du poids total de la composition de polyuréthane (P), d'au moins un agent complexant (B) ;
c) de 0,005 à 5 % en poids (50 à 50 000 ppm), sur la base du poids total de la composition de polyuréthane (P), d'au moins un composant actif de manière antimicrobienne organique (C) comprenant au moins un atome d'azote ;
d) éventuellement jusqu'à 15 % en poids, sur la base du poids total de la composition de polyuréthane (P), d'un ou plusieurs polymères (D) différents de l'au moins un composant polyuréthane (A) et
e) éventuellement jusqu'à 5 % en poids, sur la base du poids total de la composition de polyuréthane (P), d'un ou plusieurs additifs (E) ;
l'au moins un agent complexant (B) étant choisi dans le groupe constitué par des composés organiques ayant au moins deux groupements fonctionnels, parmi lesquels au moins un groupement fonctionnel est un groupement acide carboxylique ou un sel correspondant ; et
l'au moins un composé actif de manière antimicrobienne organique (C) comprenant au moins un atome d'azote étant choisi dans le groupe constitué par des sels d'ammonium quaternaire ayant au moins un groupement hydrocarboné en C₈₋₃₀ fixé à l'atome d'azote, des amines primaires, secondaires et tertiaires ayant au moins un groupement hydrocarboné en C₈₋₃₀ fixé à l'atome d'azote, des N-oxydes d'amines tertiaires ayant au moins un groupement hydrocarboné en C₈₋₃₀ fixé à l'atome d'azote, des amines tertiaires ayant un groupement hydrocarboné en C₈₋₃₀ et deux groupements aminoalkyle fixés à l'atome d'azote tertiaire, et des polymères ayant des motifs répétitifs comprenant des groupements ammonium quaternaire ou des groupements amine.

2. Composition de polyuréthane (P) selon la revendication 1, l'au moins un agent complexant (B) étant choisi dans le groupe constitué par l'acide éthylènediaminetétraacétique (EDTA) ou des sels correspondants, l'acide glycolique ou des sels correspondants, l'acide malique ou des sels correspondants, l'acide tartrique ou des sels correspondants, l'acide lactique ou des sels correspondants, l'acide citrique ou des sels correspondants, l'acide N-(1-carboxylato-éthyl)-iminodiacétique ou des sels correspondants, l'acide nitrilotriacétique ou des sels correspondants.

3. Composition de polyuréthane (P) selon la revendication 1 ou 2, l'au moins un agent complexant (B) étant choisi parmi l'acide éthylènediaminetétraacétique (EDTA) ou des sels correspondants, préférablement le sel disodique correspondant ou le sel tétrasodique correspondant.

4. Composition de polyuréthane (P) selon l'une quelconque des revendications 1 à 3, l'au moins un composant actif de manière antimicrobienne (C) étant choisi dans le groupe constitué par des sels d'ammonium quaternaire ayant au moins un groupement hydrocarboné en C₈₋₃₀ aliphatique et au moins un groupement silylalkyle alcoxylé fixés à l'atome d'azote, des N-oxydes d'amines tertiaires ayant au moins un groupement hydrocarboné en C₈₋₃₀ fixé à l'atome d'azote, des sels d'ammonium quaternaire ayant au moins deux groupements ammonium hydrocarboné en C₈₋₃₀ aliphatique fixé à l'atome d'azote, des sels d'ammonium quaternaire ayant au moins un groupement hydrocarboné en C₈₋₃₀ aliphatique et au moins un groupement hydrocarboné en C₆₋₁₄ aromatique fixés à l'atome d'azote, un poly(chlorure de diallyldiméthylammonium), un poly(dialkylamine-co-épichlorhydrine), une C₈₋₃₀ alkylamine dipropylènediamine et une poly(éthylène imine).

5. Composition de polyuréthane (P) selon l'une quelconque des revendications 1 à 4, l'au moins un composant actif de manière antimicrobienne (C) étant choisi parmi des sels d'ammonium quaternaire ayant au moins un groupement hydrocarboné en C₈₋₃₀ aliphatique et au moins un groupement silylalkyle alcoxylé fixés à l'atome d'azote, préférablement des sels de C₈₋₃₀-alkyldiméthyl(3-trialcoxysilyl)propylammonium, plus préférablement le chlorure de diméthyltétradécyl(3-(triméthoxysilyl)propyl)ammonium ou le chlorure de diméthyloctadécyl(3-(triméthoxysilyl)propyl)ammonium.

6. Composition de polyuréthane (P) selon l'une quelconque des revendications 1 à 5, l'au moins un agent complexant (B) étant présent en une quantité allant de 1 000 à 12 000 ppm, préférablement en une quantité allant de 1500 à 10 000 ppm, plus préférablement en une quantité allant de 2 000 à 8000 ppm, sur la base du poids total de la composition de polyuréthane (P).

7. Composition de polyuréthane (P) selon l'une quelconque des revendications 1 à 6, l'au moins un composant actif de manière antimicrobienne (C) comprenant au moins un atome d'azote, étant présent en des quantités de :
i) 250 à 8 000 ppm, préférablement 500 à 6 000 ppm, plus préférablement 625 à 5 000 ppm, sur la base du poids total de la composition de polyuréthane (P), et étant le chlorure de diméthyltétradécyl(3-(triméthoxysilyl)propyl)ammonium ;
ii) 250 à 4 000 ppm, préférablement 500 à 3 000 ppm, plus préférablement 625 à 2 500 ppm, sur la base du poids total de la composition de polyuréthane (P), et étant le chlorure de diméthyloctadécyl(3-(triméthoxysilyl)propyl)ammonium ;
iii) 400 à 40 000 ppm, préférablement 600 à 32 000 ppm, plus préférablement 800 à 12 000 ppm, encore plus préférablement 1 000 à 8 000 ppm, sur la base du poids total de la composition de polyuréthane (P), et étant choisi parmi les sels d'ammonium quaternaire ayant au moins deux groupements hydrocarbonés aliphatiques en C₈₋₃₀ fixés à l'atome d'azote, préférablement le chlorure de didécyldiméthylammonium ;
iv) 1 500 à 10 000 ppm, préférablement 2 000 à 8 000 ppm, plus préférablement 2 500 à 5 000 ppm, sur la base du poids total de la composition de polyuréthane (P), et étant choisi parmi les sels d'ammonium quaternaire ayant au moins un groupement hydrocarboné aliphatique en C₈₋₃₀ et au moins un groupement hydrocarboné aromatique en C₆₋₁₄ fixés à l'atome d'azote, préférablement le chlorure de C₈₋₁₈-alkyldiméthylbenzylammonium ;
v) 2 500 à 25 000 ppm, préférablement 4 000 à 12 000 ppm, plus préférablement 5 000 à 10 000 ppm, sur la base du poids total de la composition de polyuréthane (P), et étant choisi parmi les sels d'ammonium quaternaire ayant un groupement hydrocarboné en C₈₋₃₀ fixé à l'atome d'azote, préférablement le chlorure de myristyltriméthylammonium ; ou
vi) 1500 à 15 000 ppm, préférablement de 2 000 à 10 000 ppm, plus préférablement 2 500 à 8 000 ppm, sur la base du poids total de la composition de polyuréthane (P), et étant choisi parmi des C₈₋₃₀ alkylamine dipropylènediamines, préférablement la laurylamine dipropylènediamine.

8. Composition de polyuréthane (P) selon l'une quelconque des revendications 1 à 6, comprenant :
i) 1 000 à 12 000 ppm, préférablement 2 000 à 10 000 ppm, sur la base du poids total de la composition de polyuréthane (P), d'EDTA ou d'un sel correspondant en tant qu'agent complexant (B) et 1 000 à 1500 ppm, sur la base du poids total de la composition de polyuréthane (P), de chlorure de diméthyloctadécyl(3-(triméthoxysilyl)propyl)ammonium comme composant actif de manière antimicrobienne organique (C) ;
ii) 3 000 à 12 000 ppm, préférablement 5 000 à 10 000 ppm, sur la base du poids total de la composition de polyuréthane (P), d'EDTA ou d'un sel correspondant en tant qu'agent complexant (B) et 1 000 à 6 000 ppm, de préférence 2 000 à 5 000 ppm, sur la base du poids total de la composition de polyuréthane (P), de sels d'ammonium quaternaire ayant au moins un groupement hydrocarboné aliphatique en C₈₋₃₀ et au moins un groupement hydrocarboné aromatique en C₆₋₁₄ fixés à l'atome d'azote, préférablement le chlorure de C₈₋₁₈-alkyldiméthylbenzylammonium en tant que composant actif de manière antimicrobienne organique (C) ;
iii) 1 000 à 12 000 ppm, de préférence 2 000 à 10 000 ppm, sur la base du poids total de la composition de polyuréthane (P), d'EDTA ou d'un sel correspondant en tant qu'agent complexant (B) et 8 000 à 25 000 ppm, de préférence 10 000 à 20 000 ppm, sur la base du poids total de la composition de polyuréthane (P), de sels d'ammonium quaternaire ayant un groupement hydrocarboné en C₈₋₃₀ lié à l'atome d'azote, de préférence le chlorure de myristyltriméthylammonium en tant que composant actif de manière antimicrobienne organique (C) ; ou
iv) 3 000 à 12 000 ppm, de préférence 5 000 à 10 000 ppm, sur la base du poids total de la composition de polyuréthane (P), d'EDTA ou d'un sel correspondant en tant qu'agent complexant (B) et 1 500 à 15 000 ppm, de préférence 2 500 à 10 000 ppm, sur la base du poids total de la composition de polyuréthane (P), de C₈₋₃₀ alkylamine dipropylènediamine, de préférence de laurylamine dipropylènediamine en tant que composant actif de manière antimicrobienne organique (C).

9. Mousse ou revêtement de polyuréthane comprenant la composition de polyuréthane (P) selon l'une quelconque des revendications 1 à 8.

10. Article, préférablement matelas, oreiller, coussin ou pansement pour une plaie, préparé à partir de la composition de polyuréthane (P) selon l'une quelconque des revendications 1 à 8 ou de la mousse ou du revêtement selon la revendication 9.

11. Procédé pour la préparation de la composition de polyuréthane (P) selon l'une quelconque des revendications 1 à 8 comprenant les étapes :
I) fourniture d'au moins un polyol ayant au moins deux, préférablement deux ou trois groupements hydroxyle et au moins un polyisocyanate ayant au moins deux, préférablement deux groupements isocyanate ;
II) fourniture d'au moins un agent complexant (B) et d'au moins un composant actif de manière antimicrobienne organique (C) tels que décrits dans l'une quelconque des revendications 1 à 8 ;
III) éventuellement fourniture d'au moins un catalyseur pour favoriser la formation de groupements uréthane à partir des groupes hydroxyle du polyol et des groupes isocyanate du polyisocyanate ;
IV) éventuellement fourniture d'un ou plusieurs additifs (E) ;
V) mélange de l'au moins un polyol, de l'au moins un agent complexant (B) et de l'au moins un composant actif de manière antimicrobienne organique (C) pour obtenir un mélange (M) ;
VI) mélange de l'au moins un polyisocyanate avec le mélange (M) ;
VII) le fait de laisser l'au moins un polyol réagir avec l'au moins un polyisocyanate afin d'obtenir la composition de polyuréthane (P) selon l'une quelconque des revendication 1 à 8 ;
le catalyseur éventuel et/ou l'additif ou les additifs éventuels (E), s'ils sont présents, pouvant être ajoutés au mélange (M) avant, après ou conjointement avec l'au moins un polyisocyanate.

12. Utilisation d'une combinaison d'au moins un agent complexant (B) et d'au moins un composant actif de manière antimicrobienne organique (C) et éventuellement d'un ou plusieurs additifs (E) tels que décrits dans l'une quelconque des revendications 1 à 8 pour la préparation d'une composition de polyuréthane antimicrobienne.

13. Utilisation d'une combinaison d'au moins un agent complexant (B) et d'au moins un composant actif de manière antimicrobienne organique (C) et éventuellement d'un ou plusieurs additifs (E) tels que décrits dans l'une quelconque des revendications 1 à 8 pour la fourniture de propriétés antimicrobiennes sur une composition de polyuréthane.

14. Kit de composants pour la préparation d'une composition de polyuréthane ou d'un article de polyuréthane, comprenant au moins un polyol ayant au moins deux, préférablement deux ou trois groupements hydroxyle ; au moins un polyisocyanate ayant au moins deux, préférablement deux groupements isocyanate ; au moins un agent complexant (B) tel que décrit dans l'une quelconque des revendications 1 à 8 ; au moins un composant actif de manière antimicrobienne (C) tel que décrit dans l'une quelconque des revendications 1 à 8 ; et éventuellement un ou plusieurs additifs (E) tels que décrits dans l'une quelconque des revendications 1 à 8.
